# FASCICULE DE BREVET EUROPEEN

(11) **EP 4 277 512 B1**
(45) Date de publication et mention de la délivrance du brevet: **05.03.2025**
(21) Numéro de dépôt: 22700760.6
(22) Date de dépôt: 11.01.2022
(51) Int. Cl.: A61B 1/24, A61B 1/32, A61C 5/90

(54) **ECARTEUR DENTAIRE POUR PRISES DE VUES AUTONOMES, BOUCHE FERMÉE OU OUVERTE**
ZAHNÄRZTLICHER RETRAKTOR ZUR AUTONOMEN BILDGEBUNG MIT GESCHLOSSENEM ODER OFFENEM MUND
DENTAL RETRACTOR FOR AUTONOMOUS IMAGING, WITH MOUTH CLOSED OR OPEN

(30) Priorité: 12.01.2021 FR 2100236
(43) Date de publication de la demande: 22.11.2023
(73) Titulaire: Dental Monitoring, 75017 Paris (FR)
(72) Inventeur: PELLISSARD, Thomas, 94700 Maisons-Alfort (FR); LANCON, Cédric, 39600 Villers Farlay (FR); ROISIN, Louis-Charles, 75016 Paris (FR)
(74) Mandataire: Novagraaf Group
(86) Numéro de dépôt international: PCT/EP2022/050463
(87) Numéro de publication internationale: WO 2022/152707

(56) Documents cités:
- EP-A1- 3 391 810
- WO-A2-2006/014371
- FR-A1- 3 087 644
- US-A1- 2004 209 225

## Description

### Domaine technique

La présente invention concerne le domaine d'acquisition d'images dentaires et plus particulièrement un kit d'acquisition comportant un écarteur dentaire facilitant l'acquisition d'images dentaires, notamment pour la mise en œuvre d'un procédé tel que décrit dans la demande internationale PCT/EP2015/074896.

### Technique antérieure

PCT/EP2015/074896 décrit un kit d'acquisition permettant d'acquérir des photos des dents d'un utilisateur. Ce kit d'acquisition comporte :
- un écarteur dentaire destiné à être placé dans la bouche de l'utilisateur ;
- un téléphone mobile pourvu d'un appareil photo.

L'écarteur dentaire présente des rebords droit et gauche, appelés « gouttières », qui peuvent être élastiquement rapprochés l'un de l'autre pour faciliter la mise en position de service de l'écarteur dentaire, dans la bouche de l'utilisateur. L'absence de rebords supérieur et inférieur permet avantageusement à l'utilisateur d'ouvrir ou de fermer la bouche, ce qui est particulièrement utile dans les applications visées dans PCT/EP2015/074896. Cependant, certaines dents, et en particulier les incisives, restent partiellement masquées par les lèvres.

US 6,923,761 ou WO2006014371 décrivent un écarteur dentaire destiné à un usage professionnel dans le cadre d'opérations chirurgicales. Cet écarteur présente l'avantage d'écarter les lèvres latéralement, mais aussi verticalement, ce qui permet de dégager les incisives. Cependant, il est très flexible pour faciliter la mise en position de service. Il se déforme donc lorsque, en position de service, il est tiré vers la droite ou la gauche de l'utilisateur. Cette déformabilité ne permet donc pas de repousser les lèvres pour des prises de vue latérales ou occlusales, notamment pour visualiser les dents au fond de la bouche.

US 2004/0209225 décrit un écarteur dentaire destiné à être entièrement positionné dans la bouche de l'utilisateur. Cet écarteur, configuré pour un usage professionnel, permet au praticien d'accéder à différentes zones de la bouche de l'utilisateur. L'écarteur dentaire étant entièrement positionné à l'intérieur de la bouche de l'utilisateur, il ne peut cependant pas être facilement manipulé. En particulier, il est difficile de tirer l'écarteur vers la droite ou vers la gauche sans masquer une partie des dents. Par ailleurs, cet écarteur est rigide pour maintenir l'accès à l'intérieur de la bouche. En particulier, cette rigidité entrave la fermeture de la bouche pour l'acquisition de photos bouches fermée.

Enfin, la mise en position de service nécessite un contact de la bouche de l'utilisateur avec les doigts de celui qui manipule l'écarteur dentaire. Elle requiert ainsi des conditions d'hygiène parfaites. Cet écarteur dentaire est donc exclusivement réservé à une utilisation professionnelle. Il est exclu qu'il puisse être remis à l'utilisateur pour que ce dernier l'installe lui-même en position de service dans sa bouche, notamment pour prendre des images avec son téléphone mobile.

Il existe donc un besoin pour un écarteur dentaire
- qui puisse être facilement manipulé et mis en position de service dans la bouche de l'utilisateur, par l'utilisateur lui-même,
- puis qui permette, dans la position de service, l'acquisition facile d'images des dents au fond de la bouche, notamment en vues occlusales ou latérales, et d'images en bouche fermée. Un objectif de la présente invention est de répondre, au moins partiellement, à ce besoin.

### Exposé de l'invention

### Ecarteur selon l'invention

L'invention propose un écarteur dentaire selon la revendication 1.

La présence des quatre rebords est considérée comme indispensable pour écarter efficacement les lèvres, tout en autorisant une déformation de l'écarteur dentaire, notamment par torsion.

Les poignées autorisent une manipulation de l'écarteur dentaire, et en particulier sa déformation pour la mise en position de service, par l'utilisateur lui-même. Un tel écarteur dentaire est donc bien adapté à la prise de photos dentaires par l'utilisateur lui-même, de manière autonome.

**Selon un premier aspect principal de l'invention,** la structure et/ou la fixation des rebords sur la structure est (sont) conformées de manière que la force de rapprochement « horizontale » nécessaire pour rapprocher de 10 mm le rebord droit et le rebord gauche, c'est-à-dire la force minimale à exercer pour obtenir un tel rapprochement, soit supérieure à 5 N, de préférence supérieure ou égale à 6 N, de préférence supérieure ou égale à 8 N, de préférence supérieure ou égale à 10 N.

Une force de rapprochement entre deux rebords est une force de traction exercée, parallèlement au plan de l'écarteur, sur l'extrémité libre d'un des deux rebords, pour la rapprocher élastiquement de l'extrémité libre de l'autre rebord, maintenue immobile. Autrement dit, la force de rapprochement est exercée sur l'extrémité libre du premier rebord, l'extrémité libre du deuxième rebord étant maintenue immobile (et l'écarteur dentaire n'étant maintenu que par l'extrémité libre du deuxième rebord). L'extrémité libre du deuxième rebord subit donc une force de réaction de même direction, de même amplitude, mais de sens opposé à la force de rapprochement.

Lorsque l'écarteur est symétrique par rapport à un plan vertical médian passant au milieu des rebords supérieur et inférieur, la force de rapprochement horizontale rapprochant de 10 mm le rebord droit et le rebord gauche va donc produire un rapprochement de 5 mm de chacune des extrémités libres des rebords droit et gauche vers ledit plan médian.

Des essais ont montré qu'avec un écarteur du type de ceux décrits dans US 6,923,761 ou WO2006014371 cités en préambule, commercialisé par la société YDM et représenté sur la figure 17, une force de rapprochement horizontale de 2,2 N suffit à rapprocher de 10 mm les extrémités libres des rebords droit et gauche l'une de l'autre. L'écarteur testé comportait un support de langue reliant physiquement les rebords droit et gauche, mais en l'absence de support de langue, ladite force de rapprochement horizontale est encore plus faible.

Comme cela apparaîtra plus clairement dans la suite de la description, un écarteur dentaire selon le premier aspect principal de l'invention présente donc une rigidité « horizontale », ce qui permet de repousser efficacement les lèvres lorsque l'écarteur est tiré vers la droite ou vers la gauche de l'utilisateur, et facilite ainsi l'acquisition d'images des dents au fond de la bouche.

Il est donc bien adapté à l'acquisition d'images par l'utilisateur de manière autonome, c'est-à-dire sans l'aide d'un professionnel des soins dentaires, notamment avec son téléphone mobile.

**Selon un deuxième aspect principal de l'invention,** la structure et/ou la fixation des rebords sur la structure est (sont) conformées de manière que la force de rapprochement « verticale » nécessaire pour rapprocher de 10 mm le rebord supérieur du rebord inférieur soit inférieure à la force de rapprochement « horizontale » nécessaire pour rapprocher de 10 mm le rebord droit et le rebord gauche.

Comme cela apparaîtra plus clairement dans la suite de la description, un écarteur dentaire selon le deuxième aspect principal de l'invention présente ainsi une flexibilité verticale qui facilite le rapprochement des rebords supérieur et inférieur l'un de l'autre, et donc l'acquisition de photos en position bouche ouverte et bouche fermée, tout en maintenant dégagées les incisives. Un écarteur dentaire selon l'invention facilite également l'acquisition de photographies en vue occlusale et en vue latérale.

Le rapprochement vertical tient compte de la déformation de la structure, mais également d'une mobilité éventuelle du rebord supérieur et/ou du rebord inférieur par rapport à la structure, de préférence par pivotement.

Les rebords supérieur et inférieur sont de préférence fixés sur la structure de l'écarteur par des jonctions autorisant un déplacement élastique vertical des extrémités libres desdits rebords relativement à ladite structure. Cette « mobilité verticale » desdites extrémités libres par rapport à la structure permet ainsi aux lèvres supérieure et inférieure de l'utilisateur de se rapprocher l'une de l'autre lorsque l'utilisateur ferme la bouche, même si la structure est maintenue rigide. Elle facilite considérablement cette fermeture.

L'amplitude dudit déplacement élastique vertical est de préférence supérieure à 5 mm, de préférence supérieure à 10 mm et/ou inférieure à 20 mm.

De préférence, lesdites jonctions autorisent ledit déplacement élastique sous l'effet d'une force inférieure à 10 N et supérieure à 0,5 N exercée sur l'extrémité libre supérieure du rebord supérieur ou sur l'extrémité libre inférieure du rebord inférieur.

De préférence, lesdites jonctions sont cependant suffisamment rigides pour maintenir les dents de devant dégagées, de préférence sensiblement totalement dégagées dans la position bouche fermée, de préférence dans n'importe quelle position de la bouche entre la position bouche totalement ouverte et la position bouche fermée. De préférence, la frontière entre la lèvre supérieure et les dents de l'arcade supérieure, telle qu'observée à travers l'ouverture de l'écarteur, reste sensiblement immobile par rapport aux dites dents lors de la fermeture de la bouche. De préférence, la frontière entre la lèvre inférieure et les dents de l'arcade inférieure, telle qu'observée à travers l'ouverture de l'écarteur, reste sensiblement immobile par rapport aux dites dents lors de la fermeture de la bouche. Les rebords supérieur et inférieur peuvent ainsi accompagner le mouvement de fermeture de la bouche.

L'écarteur permet ainsi d'acquérir des images bouche ouverte et bouche fermée, tout en facilitant et en améliorant l'acquisition d'images en vues latérales et occlusales.

**Selon un troisième aspect principal de l'invention,** compatible avec les autres aspects principaux, l'invention propose un écarteur au moins en partie, de préférence complètement de couleur grise.

De préférence, la couleur de l'écarteur comporte, en code RGB (Red, Green, Blue) sensiblement la même valeur pour le rouge, le vert et le bleu, l'écart maximal entre les valeurs pour le rouge, le vert et le bleu étant de préférence inférieur à 10, de préférence inférieur à 5, de préférence sensiblement nul. De préférence, ces valeurs sont comprises entre 50 et 210, de préférence entre 70 et 190, de préférence encore entre 90 et 170, de préférence entre 140 et 160. Par exemple, l'écarteur peut avoir une couleur proche de (120, 120, 120), ou (150, 150,150).

De manière surprenante, les inventeurs ont découvert que l'analyse de la couleur des dents sur des photos en couleurs réalistes en est facilitée et améliorée. Un tel écarteur dentaire permet alors l'accélération du traitement des photos pour une analyse colorimétrique.

Sans être liés par cette théorie, les inventeurs expliquent ce résultat du fait que la couleur grise est une couleur neutre, qui n'altère pas les couleurs lors d'une acquisition. En effet, les réglages classiquement utilisés par les appareils d'acquisition, en particulier les appareils d'acquisition personnels comme les téléphones mobiles ou les tablettes, peuvent modifier les couleurs de la photo, notamment en rééquilibrant la balance des blancs.

De préférence, l'écarteur est de couleur grise au moins dans la ou les régions susceptibles d'être visibles sur les images acquises dans la position de service, notamment lorsque l'appareil d'acquisition d'images, en particulier un téléphone mobile, est solidarisé à l'écarteur au moyen d'un support.

Un écarteur dentaire selon le premier et/ou le deuxième et/ou troisième aspect principal de l'invention peut comporter une ou plusieurs des caractéristiques optionnelles suivantes :
- l'écarteur dentaire est configuré de manière à être entièrement du côté extérieur des dents dans la position de service, de manière à autoriser une occlusion complète ;
- l'écarteur dentaire est configuré de manière à laisser entièrement dégagée la vue à travers l'ouverture d'écarteur dans la position de service, de préférence est dépourvu de support de langue ;
- l'écarteur dentaire comporte une poignée s'étendant à l'extérieur de la bouche de l'utilisateur dans la position de service ;
- la poignée présente la forme d'une patte, de préférence orientée vers le centre de l'ouverture d'écarteur ;
- la poignée est conformée pour être écartée de la surface extérieure des joues de l'utilisateur dans la position de service ;
- la poignée est fixée sur une des extrémités droite et gauche de la structure et/sur un des rebords droit et gauche ;
- l'écarteur dentaire comporte desdites poignées droite et gauche rigidement fixées aux extrémités droite et gauche de la structure, respectivement, et/ou aux rebords droit et gauche, respectivement ;
- les poignées droite et gauche sont conformées pour un positionnement de l'écarteur dentaire dans ladite position de service, sans contact avec la bouche de l'utilisateur, c'est-à-dire autorisent une mise de l'écarteur dentaire dans la position de service sans contact des doigts manipulant l'écarteur dentaire avec la bouche de l'utilisateur ;
- les poignées droite et gauche, de préférence sous la forme de pattes, sont agencées de manière à s'étendre radialement vers l'extérieur de l'ouverture d'écarteur ;
- chaque rebord présente une longueur, mesurée en suivant ledit rebord, supérieure à 1 cm, de préférence supérieure à 2 cm et/ou inférieure à 15 cm, de préférence inférieure à 12 cm, de préférence inférieure à 8 cm ;
- le rapport de ladite force de rapprochement horizontale sur ladite force de rapprochement verticale est supérieur à 2, de préférence supérieur à 4, de préférence supérieur à 4, de préférence supérieur à 5, de préférence supérieur à 6, de préférence supérieur à 7 et/ou inférieur à 15, de préférence inférieur à 12, de préférence inférieur à 10 ;
- ladite force de rapprochement horizontale est inférieure à 20 N, à 15 N, et/ou supérieure à 5 N, de préférence supérieure ou égale à 6 N, de préférence supérieure ou égale à 8 N, de préférence supérieure ou égale à 10 N ;
- ladite force de rapprochement verticale est inférieure à 8 N, à 6 N, à 4 N, à 3 N, à 2 N, de préférence inférieure ou égale à 1,5 N, et/ou supérieure à 0,5 N, de préférence supérieure à 1 N ;
- la structure est conformée de manière qu'une déformation de la structure entraînant un mouvement des rebords droit et gauche l'un par rapport à l'autre entraîne un mouvement des rebords supérieur et inférieur l'un par rapport à l'autre, lesdits rebords droit et gauche se rapprochant de préférence lorsque les rebords supérieur et inférieur s'écartent, et réciproquement ;
- la structure est conformée de manière qu'une déformation de la structure entraînant un mouvement des points de fixation des rebords supérieur et inférieur l'un de l'autre entraîne un mouvement des rebords droit et gauche l'un par rapport à l'autre, lesdits points de fixation se rapprochant de préférence lorsque les rebords droit et gauche s'écartent, et réciproquement;
- la structure comporte au moins un arceau, les rebords droit et gauche étant fixés, de préférence rigidement, à des extrémités droite et gauche dudit arceau, respectivement, de sorte que ledit arceau s'oppose élastiquement à un rapprochement des rebords droit et gauche l'un de l'autre ;
- l'écarteur comporte deux dits arceaux, dits arceaux supérieur et inférieur, de préférence coplanaires, de préférence de même forme générale, de préférence symétriques, sur lesquels sont fixés les rebords supérieur et inférieur, respectivement ;
- les arceaux supérieur et inférieur se rejoignent en leurs extrémités de manière que la structure présente une forme globalement ovale, similaire au contour d'un ballon de rugby ;
- les arceaux supérieur et inférieur sont configurés pour se rapprocher de 10 mm sous l'action d'une force de rapprochement verticale desdits arceaux inférieure à 15 N, à 10 N, à 8 N, de préférence inférieure ou égale à 6 N, et/ou supérieure à 3 N, de préférence supérieure à 4 N, la force de rapprochement verticale entre les deux arceaux étant une force de traction exercée, parallèlement au plan de l'écarteur, au milieu d'un desdits arceaux pour le rapprocher élastiquement du milieu de l'autre arceau, maintenu immobile ;
- les arceaux supérieur et inférieur sont configurés pour se rapprocher de 10 mm sous l'action d'une force de rapprochement horizontale desdits arceaux, inférieure à 20 N, à 15 N, et/ou supérieure à 5 N, de préférence supérieure ou égale à 6 N, de préférence supérieure ou égale à 8 N, de préférence supérieure ou égale à 10 N, la force de rapprochement horizontale entre les deux arceaux étant une force de traction exercée, horizontalement et parallèlement au plan de l'écarteur, sur une jonction entre lesdits arceaux pour la rapprocher élastiquement de l'autre jonction entre lesdits arceaux, maintenue immobile ;
- de manière plus générale, de préférence, la structure n'est comprimable horizontalement de 10 mm que sous l'action d'une force de rapprochement horizontale F_{H} des deux extrémités latérales (extrémités droite et gauche) de la structure supérieure à 5 N, de préférence supérieure ou égale à 6 N, de préférence supérieure ou égale à 8 N, de préférence supérieure ou égale à 10 N ;
- le rapport de ladite force de rapprochement horizontale des arceaux sur ladite force de rapprochement verticale des arceaux est supérieur à 1, de préférence supérieur à 1,5, de préférence supérieur à 2, de préférence supérieur à 2,3, et/ou inférieur à 5, de préférence inférieur à 3, de préférence inférieur à 2,5 ;
- le rebord supérieur et/ou le rebord inférieur est (sont) montés pivotants élastiquement sur les arceaux supérieur et inférieur, respectivement, de préférence autour d'axes horizontaux dans la position de service, ledit pivotement du rebord supérieur et/ou du rebord inférieur autorisant un déplacement vertical de l'extrémité libre du rebord supérieur et/ou du rebord inférieur, respectivement, par rapport à l'arceau supérieur et/ou à l'arceau inférieur respectivement, d'une distance de 10 mm sous l'effet d'une force inférieure à 10 N, 5 N, 3 N, 2 N, de préférence inférieure ou égale à 1,5 N, et/ou supérieure à 0,5 N, de préférence supérieure à 1 N ;
- au moins un des rebords supérieur et inférieur est venu de matière avec ledit arceau supérieur ou inférieur, respectivement ;
- la structure est conformée de manière à s'étendre radialement à l'extérieur de la bouche de l'utilisateur dans la position de service ;
- les arceaux supérieur et inférieur sont agencés de manière à s'étendre à l'extérieur de la bouche de l'utilisateur dans la position de service ;
- les arceaux supérieur et inférieur sont agencés de manière à s'étendre radialement à l'extérieur de l'ouverture d'écarteur dans la position de service, c'est-à-dire que lorsque l'écarteur est observé suivant l'axe de ladite ouverture, ladite ouverture apparaît incluse dans la surface définie par lesdits arceaux ;
- le rebord supérieur et/ou le rebord inférieur comporte(nt) des ailettes droite et gauche configurées pour entrer en butée avec les rebords droit et gauche, respectivement, de manière à entraver l'écartement des rebords supérieur et inférieur l'un de l'autre lors d'un un rapprochement des rebords droit et gauche l'un de l'autre ;
- en tout point d'un dit arceau supérieur et inférieur, la pente de l'arceau est supérieure ou égale à 0° et inférieure ou égale à 60°, de préférence inférieure à 50°, de préférence inférieure à 45°, de préférence inférieure à 40°, la pente étant la valeur absolue du plus petit angle formé par une tangente audit arceau audit point, avec un plan horizontal dans la position de service, c'est-à-dire avec un plan perpendiculaire au plan général de l'écarteur et contenant les centres des rebords droit et gauche ;
- la pente maximale dudit arceau le long dudit arceau est supérieure ou égale à 20°, de préférence supérieure à 30°, et/ou inférieure ou égale à 60°, de préférence inférieure à 50°, de préférence inférieure à 45°, de préférence inférieure à 40° ;
- la pente dudit arceau augmente continument, de préférence de manière régulière, depuis le milieu dudit arceau vers l'une quelconque de ses deux extrémités ;
- au moins un des rebords supérieur et inférieur comporte une encoche de réception d'un frein labial ;
- l'encoche présente une forme arrondie, rectangulaire, carrée, ou triangulaire ;
- le rebord supérieur et le rebord inférieur comporte chacun une encoche, l'encoche du rebord supérieur et l'encoche du rebord inférieur étant de préférence identiques ;
- l'encoche présente une largeur supérieure à 3 mm, 5 mm, 8 mm, 10 mm, la largeur étant mesurée à l'ouverture de ladite encoche.

### Kit selon l'invention

L'invention propose encore un kit comportant :
- un écarteur dentaire, de préférence selon le premier et/ou le deuxième et/ou le troisième aspect principal de l'invention ; et
- un support d'écarteur, sélectivement indépendant de l'écarteur, dans une position désassemblée, ou fixé sur l'écarteur, dans une position assemblée.

**Selon un quatrième aspect principal de l'invention,** dans une position assemblée, la fixation du support sur l'écarteur est configurée pour imposer une distance entre les rebords droit et gauche.

Comme cela apparaîtra plus clairement dans la suite de la description, l'assemblage du support d'écarteur et de l'écarteur permet de figer ladite distance entre les rebords droit et gauche, et donc de rigidifier plus encore la structure de l'écarteur. Le support d'écarteur agit comme une clé de verrouillage qui fige la configuration de la structure de l'écarteur. Dans la position assemblée, la structure de l'écarteur peut être ainsi qualifiée de « verrouillée ».

Le verrouillage de la structure de l'écarteur facilite la transmission d'efforts sur les lèvres lorsque l'écarteur est déplacé latéralement ou verticalement par rapport à l'utilisateur, dans la position de service. Les lèvres sont ainsi efficacement repoussées, ce qui facilite en particulier la prise de vue latérale et occlusale pour l'acquisition de photos de dents au fond de la bouche.

Le verrouillage de la structure de l'écarteur permet également d'améliorer le dégagement des dents de devant (incisives, canines), notamment lorsque l'utilisateur a la bouche ouverte. Dans un mode de réalisation préféré, les extrémités libres des rebords supérieur et inférieur peuvent néanmoins se rapprocher élastiquement l'une de l'autre, y compris dans la position assemblée de l'écarteur. L'utilisateur peut avantageusement fermer la bouche sans être trop gêné par la rigidité de la structure de l'écarteur. L'élasticité du rapprochement desdites extrémités libres permet cependant de conserver un dégagement des dents de devant.

Dans la position désassemblée, la déformabilité de la structure, c'est-à-dire sa capacité à être déformé à la main par l'utilisateur, facilite en particulier la mise en position de service de l'écarteur dentaire par l'utilisateur, notamment grâce à une torsion et/ou une flexion de l'écarteur dentaire permettant d'insérer l'écarteur dans la bouche de l'utilisateur.

De préférence, la structure est configurée de manière que, dans la position désassemblée, les rebords droit et gauche puissent être rapprochés élastiquement l'un par rapport à l'autre et les rebords supérieur et inférieur puissent être rapprochés élastiquement l'un par rapport à l'autre. La mise en position de service, dans la bouche de l'utilisateur, en est encore facilitée.

De préférence, le support d'écarteur peut être fixé sur l'écarteur alors que l'écarteur est dans la position de service. Il s'étend de préférence à l'extérieur de la bouche dans la position de service.

La rigidité de la structure avant verrouillage permet avantageusement aux parties d'attache de l'écarteur d'être sensiblement écartées de la même distance que les parties d'attache du support d'écarteur correspondantes. L'assemblage de l'écarteur au support en est avantageusement grandement simplifié.

**Selon un cinquième aspect principal de l'invention,** compatible avec les autres aspects principaux, le kit est remarquable en ce que, dans la position assemblée, chaque partie d'attache d'écarteur est plaquée sur une partie d'attache de support correspondante, de préférence attirée magnétiquement par une partie d'attache de support correspondante, suivant un axe d'attachement, et en ce que lesdites parties d'attache d'écarteur et de support sont configurées pour bloquer par butée un déplacement, de préférence tout déplacement dans un plan perpendiculaire à l'axe d'attachement.

De préférence, les parties d'attache d'écarteur et de support sont configurées pour, dans la position de service, bloquer par butée au moins un déplacement du support relativement à l'écarteur vers la droite de l'utilisateur, vers la gauche de l'utilisateur, vers le haut ou vers le bas, en particulier de manière qu'en position de service, lorsque l'utilisateur déplace l'écarteur vers sa droite ou vers sa gauche en agissant sur le support, le support et l'écarteur restent attachés l'un à l'autre.

Comme cela apparaîtra plus clairement dans la suite de la description, l'assemblage du support d'écarteur et de l'écarteur peut être ainsi avantageusement simple à réaliser et fiable. En particulier, avec des attaches magnétiques, il suffit de rapprocher chaque partie d'attache d'écarteur de la partie d'attache de support correspondante pour assurer un plaquage selon la direction de l'attraction magnétique. Le plaquage conduit à disposer la butée dans sa position de blocage, ce qui évite ensuite un détachement par traction dans un plan perpendiculaire à cette direction.

Un kit selon le quatrième et/ou le cinquième aspect principal de l'invention peut comporter une ou plusieurs des caractéristiques optionnelles suivantes :
- l'écarteur comporte des parties d'attache d'écarteur et ledit support d'écarteur comporte des parties d'attache de support disposées de manière à coopérer, de préférence magnétiquement, avec lesdites parties d'attache d'écarteur de manière à fixer, de manière amovible, optionnellement de manière rigide, ledit écarteur sur ledit support d'écarteur ;
- le support d'écarteur est rigide entre les parties d'attaches de support, de sorte qu'après fixation de l'écarteur sur le support d'écarteur, lesdites parties d'attache d'écarteur ne peuvent être écartées ni rapprochées l'une de l'autre ;
- au moins des parties d'attache d'écarteur droite et gauche sont rigidement solidaires des rebords droit et gauche, respectivement ;
- les parties d'attaches d'écarteur sont fixées aux jonctions entre les arceaux inférieur et supérieur ;
- l'écarteur et le support d'écarteur sont fixés l'un à l'autre, dans la position assemblée, par un ou plusieurs clips, des bandes auto-agrippantes, par exemple de type Velcro^{®}, des mâchoires de serrage, des vis, des aimants, une complémentarité de forme entre le support d'écarteur et l'écarteur, des moyens d'attache élastique, par exemple une bande élastique, des bandes auto-collantes, des crochets, ou des ventouses ;
- de préférence, le kit comporte encore un appareil d'acquisition d'images, de préférence un téléphone mobile, fixé sur le support d'écarteur, de préférence de manière amovible, de manière à acquérir des images représentant au moins partiellement l'ouverture d'écarteur.

Un kit selon l'invention comporte de préférence un écarteur dentaire selon le premier et/ou deuxième et/ou troisième aspect principal.

### Procédé selon l'invention

L'invention concerne également un procédé d'acquisition d'images dentaires au moyen d'un écarteur dentaire ou d'un kit selon l'invention, comportant les étapes suivantes :
a) mise en position de service de l'écarteur dentaire, en position désassemblée dans le cas où le procédé est mis en œuvre au moyen d'un kit selon l'invention ;
b) dans le cas où le procédé est mis en œuvre au moyen d'un kit selon l'invention, après l'étape a), fixation du support d'écarteur à l'écarteur dentaire de façon que l'écarteur dentaire soit en position assemblée ;
c) déplacement de l'écarteur dentaire, de préférence en faisant faire un mouvement de rotation à l'écarteur dentaire autour de l'utilisateur, vers la droite et/ou vers la gauche de l'utilisateur, de façon à rendre au moins une molaire visible à travers l'ouverture d'écarteur, de préférence de manière que l'ouverture d'écarteur X soit sensiblement en face de ladite molaire, et/ou
   fermeture et/ou ouverture de la bouche de l'utilisateur de façon à rendre visibles, à travers l'ouverture d'écarteur, des dents en position bouche fermée ou en position bouche ouverte, respectivement ;
d) de préférence, acquisition, au moyen de l'appareil d'acquisition, d'au moins une image représentant ladite molaire et/ou représentant des dents en position bouche fermée ou bouche ouverte.

L'étape a) comporte notamment la déformation de l'écarteur dentaire, en exerçant une force sur les rebords droit, gauche, supérieur et/ou inférieur, de façon à modifier l'ouverture d'écarteur et faciliter l'insertion de l'écarteur dans la bouche de l'utilisateur.

L'étape a) comporte de préférence une torsion de l'écarteur dentaire selon un axe horizontal et/ou d'une flexion de l'écarteur dentaire selon un axe vertical. Une torsion ou une flexion de la structure permet ainsi avantageusement d'introduire facilement l'écarteur dentaire dans la bouche de l'utilisateur, en dépit de la rigidité horizontale.

Avantageusement, la présence d'ailettes droite et gauche sur le rebord supérieur et/ou inférieur permet de limiter l'écartement vertical dudit rebord supérieur et dudit rebord inférieur lorsque les rebords droit et gauche sont rapprochés pour positionner l'écarteur dentaire en position de service.

Avant l'étape d), de préférence avant l'étape c), de préférence avant l'étape b), de préférence encore avant l'étape a), l'appareil d'acquisition d'images est fixé sur le support d'écarteur, de telle sorte que l'objectif de l'appareil d'acquisition d'images soit en face de l'ouverture d'écarteur. Cette position permet ainsi à l'appareil d'acquisition d'images d'acquérir une image représentant l'ouverture d'écarteur, et donc des images de l'intérieur de la bouche dans la position de service.

Dans un mode de réalisation particulier, l'appareil d'acquisition d'images est indissociable du support d'écarteur. Par exemple, le support d'écarteur peut comporter un appareil photo ou une caméra intégrée, dont l'objectif fait face à l'ouverture d'écarteur.

De préférence, l'appareil d'acquisition d'images est fixé sur le support d'écarteur, de préférence de manière amovible, de sorte que son axe optique croise toujours l'ouverture d'écarteur, quelle que soit la position l'appareil d'acquisition d'images sur le support d'écarteur.

A l'étape c), le déplacement de l'écarteur dentaire peut être effectué grâce au déplacement du support d'écarteur, lorsque l'écarteur dentaire est fixé au support d'écarteur.

De préférence, les étapes c) et d), sont répétées plusieurs fois, par exemple en effectuant une première rotation vers la droite afin d'acquérir une photo des molaires droites, puis en effectuant une deuxième rotation vers la gauche afin d'acquérir une photo des molaires gauches. L'écarteur dentaire peut également être déplacé vers le haut et/ou vers le bas afin d'acquérir des images de vues occlusales. L'utilisateur peut alternativement ou additionnellement acquérir une ou des photos bouche ouverte puis bouche fermée ou inversement.

Une étape, de préférence toutes les étapes, peuvent être effectuées par l'utilisateur lui-même ou un de ses proches.

Des instructions visuelles et/ou sonores peuvent être générées. En particulier, des instructions peuvent indiquer à l'utilisateur comment positionner l'écarteur dentaire en position de service, en particulier comment déformer l'écarteur dentaire pour faciliter sa mise en position de service, comment fixer le support d'écarteur à l'écarteur dentaire et/ou à l'appareil d'acquisition d'images au support d'écarteur pour l'utilisation d'un kit selon l'invention, combien d'images il doit acquérir et/ou les déplacements à effectuer pour acquérir différentes vues. Les instructions peuvent être émises par l'appareil d'acquisition d'images.

L'invention concerne également :
- un programme informatique, ou « applicatif », et en particulier un applicatif spécialisé pour téléphone mobile, comprenant des instructions de code de programme pour guider une prise de vues, et en particulier, de préférence, pour
   - préciser à un utilisateur le nombre d'images qu'il doit acquérir, et/ou
   - guider un utilisateur lors d'un réglage de la géométrie du support d'écarteur et/ou d'un positionnement de la bouche de l'utilisateur sur l'écarteur dentaire, et/ou du positionnement de l'écarteur dentaire par rapport à la bouche de l'utilisateur et/ou des déplacements à effectuer pour acquérir les images ;
- un support informatique sur lequel est enregistré un tel programme, par exemple une mémoire ou un CD-ROM, et
- un appareil personnel, en particulier un téléphone mobile ou une tablette, dans lequel est chargé un tel programme.

### Définitions

Par « utilisateur », on entend toute personne pour laquelle un écarteur dentaire ou un kit selon l'invention peut être mis en œuvre, que cette personne soit malade ou non, ou que cette personne soit en cours de traitement ou non. Un écarteur selon l'invention peut être utilisé pour un autre animal qu'un être humain.

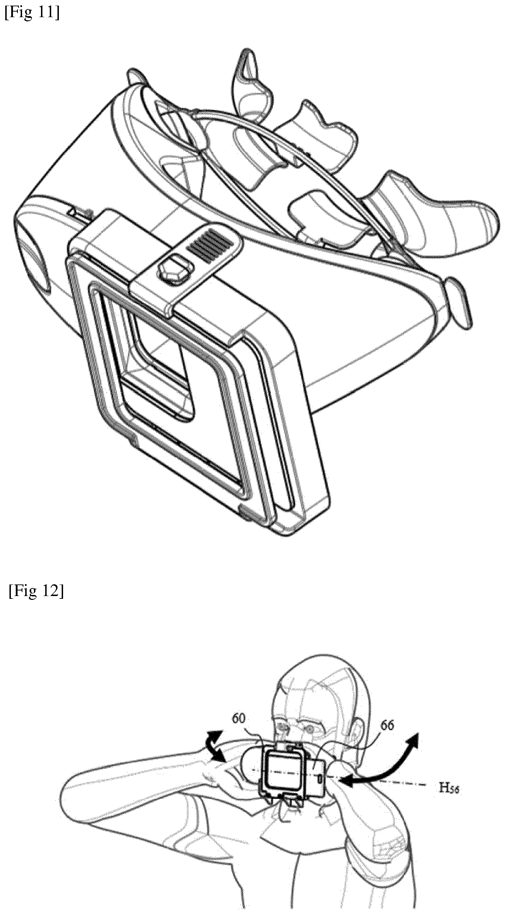

La position de service est la position dans laquelle l'écarteur est disposé sur la bouche d'un utilisateur maintenant sa tête verticalement, comme illustré sur la figure 12.

La position « bouche fermée » est la position d'occlusion dans laquelle les dentures supérieure et inférieure sont en contact.

La position « bouche ouverte » est la position d'ouverture totale de la bouche.

Le plan de l'écarteur est le plan de l'ouverture qu'il définit.

Un « support de langue » est classiquement une partie d'un écarteur dentaire qui pénètre à l'intérieur de la bouche de manière à interagir avec la langue de l'utilisateur dans la position de service, en particulier pour lui servir de support ou pour l'écarter.

On appelle "téléphone mobile" un appareil de moins de 500 g, doté d'un capteur lui permettant de capturer des images, capable d'échanger des données avec un autre appareil éloigné de plus de 500 km du téléphone mobile, et capable d'afficher lesdites données, et notamment lesdites images.

Deux pièces sont « mobiles élastiquement » l'une par rapport à l'autre, si leur position relative peut être modifiée sous l'action d'une force et si, lorsque cette force cesse, elles retrouvent leurs positions relatives initiales.

Les rebords droit et gauche sont des rebords « opposés ». Il en est de même des rebords supérieur et inférieur. Un écarteur dentaire selon l'invention comporte donc deux couples formés de deux rebords opposés, à savoir le couple des rebords droit et gauche et le couple des rebords supérieur et inférieur.

La rigidité de la structure n'est pas incompatible avec la présence de rebords mobiles, en particulier mobiles élastiquement. En particulier, les rebords sont considérés comme étant mobiles. Si les rebords ne sont pas mobiles l'un par rapport à l'autre alors l'écarteur comporte une rigidité totale ou une rigidité verrouillée. En particulier, la fixation du support d'écarteur à l'écarteur dentaire permet une rigidité verrouillée horizontale.

Une « force de rapprochement » est une force exercée sur un premier rebord, dans la direction du rebord opposé audit premier rebord, alors que le rebord opposé est immobilisé, de manière à pousser le premier rebord vers le bord opposé.

Les « conditions d'acquisition » d'une image des dents précisent la position et l'orientation dans l'espace d'un appareil d'acquisition d'images relativement aux dents de l'utilisateur, et de préférence la calibration de cet appareil d'acquisition d'images, pour acquérir à l'instant de ladite acquisition, par observation directe, ladite image.

Par "image", on entend une image en deux dimensions, comme une photographie. Une image est formée de pixels.

Sauf indications contraires, les qualificatifs utilisés pour définir des positions dans l'espace comme « horizontal », « vertical », « bas », « haut », « droite » ou « gauche » sont définies, à des fins de clarté, en référence à une position de service observée par un utilisateur, ayant la tête droite et ayant disposé ses lèvres sur l'écarteur dentaire.

Dans la position de service, l'axe longitudinal X est l'axe qui s'étend perpendiculairement au plan général de l'ouverture d'écarteur et qui passe par le centre de l'ouverture d'écarteur.

« Fixer » une pièce sur une autre signifie établir un lien physique entre ces deux pièces, de sorte que tout déplacement de l'une de ces pièces finit par entrainer celui de l'autre pièce. Une pièce montée en translation, à rotation ou à rotule sur une autre pièce est fixée à cette autre pièce. Une fixation peut être « définitive » ou « de manière amovible », selon que les deux pièces sont adaptées ou non à une désolidarisation, de préférence à la main, par un utilisateur. Une fixation peut être rigide ou non, selon qu'elle autorise ou non un déplacement relatif des deux pièces fixées l'une à l'autre.

"Comprendre", "comporter" et "présenter" doivent être interprétés de manière large et non limitative, sauf indication contraire.

### Brève description des dessins

D'autres caractéristiques et avantages de l'invention apparaîtront encore à la lecture de la description détaillée qui va suivre et à l'examen du dessin annexé dans lequel :
[Fig 1] la figure 1 représente un écarteur dentaire selon l'invention, en perspective, représenté du côté opposé au côté destiné à être positionné dans la bouche d'un utilisateur en position de service ;
[Fig 2] la figure 2 représente l'écarteur dentaire de la figure 1, en perspective, représenté du côté destiné à être positionné dans la bouche d'un utilisateur en position de service ;
[Fig 3] la figure 3 représente l'écarteur dentaire de la figure 1 représenté vu de dessus (3A), de devant (3B), de dessous (3C) et de derrière (3D) ;
[Fig 4] la figure 4 représente un autre écarteur dentaire selon l'invention représenté du côté destiné à être positionné dans la bouche d'un utilisateur en position de service, et en perspective ;
[Fig 5] la figure 5 est un exemple de photographie d'une vue occlusale avec un écarteur dentaire conventionnel en position de service ;
[Fig 6] la figure 6 est un autre exemple de photographie d'une vue occlusale avec un écarteur dentaire conventionnel en position de service ;
[Fig 7] la figure 7 est un exemple de photographie d'une vue de face comportant un écarteur dentaire selon l'invention en position de service ;
[Fig 8] la figure 8 est un exemple de photographie d'une vue latérale comportant un écarteur dentaire selon l'invention en position de service ;
[Fig 9] la figure 9 est un exemple de photographie d'une vue occlusale comportant un écarteur dentaire selon l'invention en position de service ;
[Fig 10] la figure 10 représente, en perspective, un kit selon l'invention, l'écarteur dentaire étant en position assemblée ;
[Fig 11] la figure 11 représente, selon une autre perspective, un kit selon l'invention, l'écarteur dentaire étant en position assemblée ; et
[Fig 12] la figure 12 représente, l'utilisation d'un kit selon l'invention, en position de service ;
[Fig 13] la figure 13 illustre la déformabilité de la structure d'un écarteur selon l'invention ;
[Fig 14] la figure 14 illustre la déformabilité du rebord supérieur d'un écarteur selon l'invention ;
[Fig 15] la figure 15 représente, sur la partie gauche, en perspective, un support d'écarteur d'un kit selon l'invention, et sur la partie droite, une coupe illustrant l'attachement de l'écarteur dentaire au dit support d'écarteur ;
[Fig 16] la figure 16 illustre la déformabilité de rebords supérieur et inférieur ; et
[Fig 17] la figure 17 représente un écarteur ayant fait l'objet d'essais comparatifs.

Dans les différentes figures, les organes identiques ou analogues ont été désignés avec des références identiques.

### Description détaillée

### Ecarteur dentaire

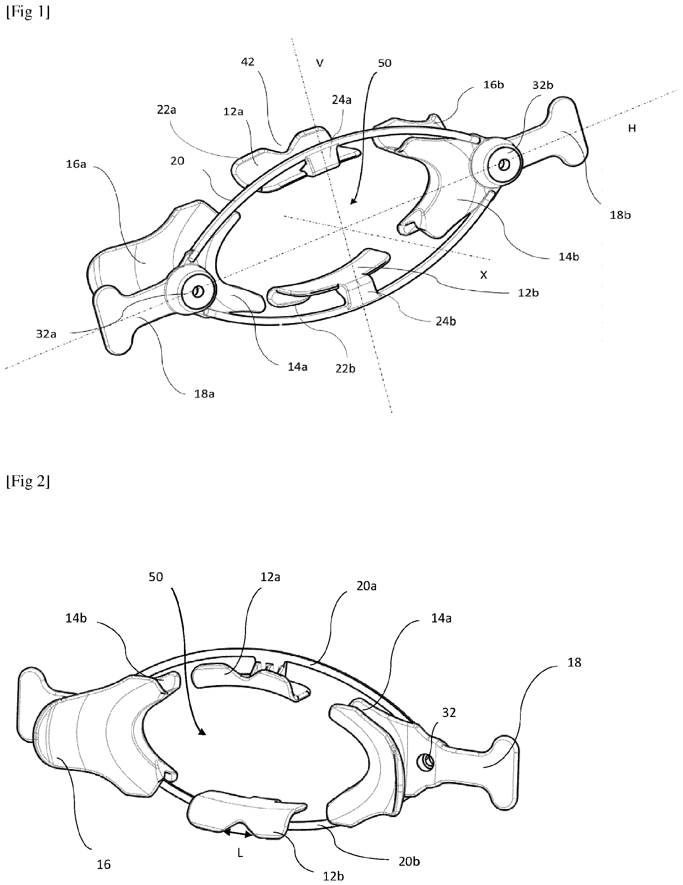

L'écarteur dentaire 10 représenté sur les figures 1, 2 et 3 est symétrique par rapport à un plan médian qui, dans la position de service s'étend verticalement (les directions verticale et horizontale étant référencées « V » et « H », respectivement).

Il comporte des rebords droit 14a (à droite de l'utilisateur dans la position de service), gauche 14b, supérieur 12a et inférieur 12b, et une structure 20 ceinturant une ouverture d'écarteur 50 comportant, dans l'exemple représenté, deux arceaux supérieur 20a et inférieur 20b.

L'ouverture d'écarteur est entièrement dégagée, c'est-à-dire que lorsqu'on observe l'écarteur selon l'axe X, aucune partie de l'écarteur n'obture, même partiellement, l'ouverture d'écarteur. L'écarteur dentaire est ainsi configuré de manière à laisser entièrement dégagée la vue à travers l'ouverture d'écarteur dans la position de service, ce qui évite avantageusement tout masquage d'une dent.

En particulier, l'écarteur dentaire est dépourvu d'une traverse, par exemple une traverse horizontale rejoignant les rebords droit et gauche ou les extrémités droite et gauche de la structure, et masquant partiellement ladite vue. Notamment, l'écarteur dentaire est de préférence dépourvu de support de langue. Avantageusement, l'écarteur peut être déformé plus facilement pour être mis en position de service.

De manière préférée, l'écarteur ne comporte aucune partie s'étendant, dans la position de service, du côté de l'intrados des dents. Autrement dit, dans la position de service et dans la position fermée de la bouche, l'écarteur ne pénètre pas dans la cavité buccale délimitée partiellement par les intrados des dents. Dans la position de service, il s'étend exclusivement à l'extérieur de la bouche et dans l'espace défini par l'extrados des dents et les lèvres. L'utilisateur peut donc fermer la bouche jusqu'à l'occlusion.

Lesdits rebords droit 14a, gauche 14b, supérieur 12a et inférieur 12b sont fixés sur ladite structure 20 à droite, à gauche, au-dessus et en-dessous de ladite ouverture d'écarteur 50, respectivement, la droite et la gauche étant définies par rapport à une situation où l'on observe l'écarteur dentaire du côté destiné à être positionné dans la bouche de l'utilisateur.

Plus précisément, dans l'exemple représenté sur les figures 1 à 3, les rebords supérieur 12a et inférieur 12b sont fixés au milieu des arceaux supérieur 20a et inférieur 20b, respectivement.

Les extrémités droites des arceaux supérieur 20a et inférieur 20b se rejoignent en une jonction droite. Les extrémités gauches des arceaux supérieur 20a et inférieur 20b se rejoignent en une jonction gauche. Les jonctions droite et gauche définissent ainsi les extrémités droite et gauche de la structure, respectivement.

Les arceaux forment ensemble un anneau aplati, dont la forme est similaire au contour d'un ballon de rugby. La pente α des arceaux, par rapport à un plan horizontal, est de préférence maximale au niveau des jonctions droite et gauche, et de préférence nulle sensiblement au milieu des arceaux.

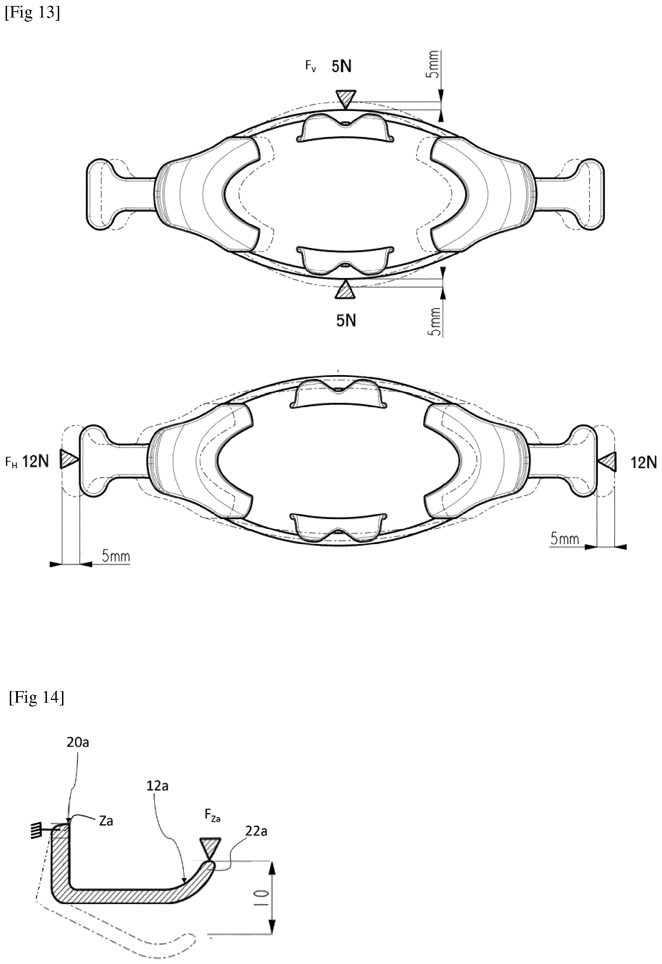

Comme illustré sur la figure 13, l'écarteur est déformable sous l'effet de forces verticale Fv ou horizontale F_{H}. Sur la figure 13, les forces F_{V} et F_{H} sont exercées au milieu de l'arceau supérieur et à la jonction gauche entre les arceaux, respectivement, et sont donc des forces de rapprochement « des arceaux ».

De manière générale, la structure est relativement rigide horizontalement. Elle n'est de préférence comprimable horizontalement de 10 mm que sous l'action d'une force de rapprochement horizontale F_{H} des deux extrémités latérales (extrémités droite et gauche) de la structure, supérieure à 5 N, de préférence supérieure ou égale à 6 N, de préférence supérieure ou égale à 8 N, de préférence supérieure à 10 N.

Dans le mode de réalisation illustré, la force de rapprochement horizontale F_{H} pour rapprocher horizontalement les rebords droit et gauche l'un de l'autre de 10 mm, en agissant exclusivement sur les jonctions des arceaux, est de préférence inférieure à 20 N, à 15 N, et/ou supérieure à 5 N, de préférence supérieure ou égale à 6 N, de préférence supérieure ou égale à 8 N, de préférence supérieure ou égale à 10 N. Sur la figure 13, elle est de 12 N.

De préférence, la force de rapprochement verticale F_{V} pour rapprocher horizontalement les rebords supérieur et inférieur l'un de l'autre de 10 mm, en agissant exclusivement sur le milieu des arceaux, est inférieure à 15 N, à 13 N, à 10 N, à 8 N, de préférence inférieure ou égale à 5 N, et/ou supérieure à 3 N, de préférence supérieure à 4 N. Sur la figure 13, elle est de 5 N.

Les rebords supérieur 12a et inférieur 12b peuvent être fixés rigidement sur la structure 20. De préférence, comme représenté sur la figure 14, au moins un des rebords supérieur 12a et inférieur 12b, de préférence chacun des rebords supérieur 12a et inférieur 12b est fixé sur l'arceau supérieur ou à l'arceau inférieur, respectivement, de manière à pourvoir se déplacer par rapport à l'arceau supérieur et à l'arceau inférieur, de préférence pivoter élastiquement, respectivement, autour d'un axe « supérieur » Za ou « inférieur », respectivement, horizontal dans la position de service. Le confort en est amélioré.

De préférence, cette élasticité permet à l'extrémité libre supérieure 22a du rebord supérieur 12a ou à l'extrémité libre inférieure 22b du rebord inférieur 12b, respectivement de se déplacer verticalement d'une distance de 10 mm sous l'effet d'une force (référence F_{Za} sur la figure 14), inférieure à 10 N, 5 N, 3 N, 2 N, de préférence inférieure ou égale à 1,5 N, et/ou supérieure à 0,5 N, de préférence supérieure à 1 N. Pour mesurer ce déplacement, la structure est maintenue rigide. La force F_{Za} résulte donc exclusivement en un déplacement, de préférence par pivotement, du rebord supérieur 12a ou du rebord inférieur 12b, respectivement (Figure 14).

Dans une mode de réalisation préféré, les jonctions supérieure 24a et inférieure 24b des rebords supérieur 12a et inférieur 12b forment des liaisons pivots, de préférence autour d'un axe horizontal dans la position de service. Le pivotement des rebords supérieur 12a et inférieur 12b permet avantageusement de rapprocher les lèvres lorsque l'utilisateur ferme la bouche.

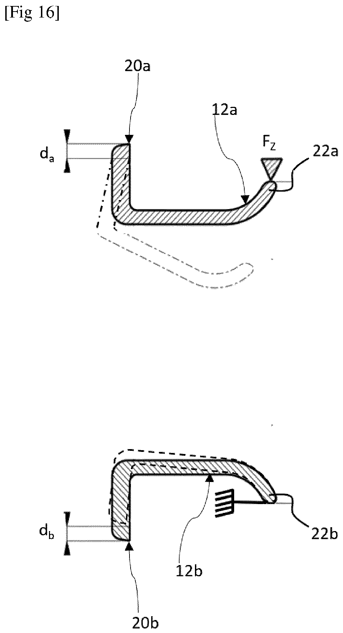

Dans un mode de réalisation, comme illustré sur la figure 16, la structure est également déformable de manière que, lorsque l'écarteur n'est pas fixé au support, un pincement pour rapprocher verticalement l'extrémité libre supérieure 22a de l'extrémité libre inférieure 22b d'une distance de 20 mm, produise également un rapprochement vertical des arceaux supérieur et inférieur d'une distance *d* (=dₐ+d_{b}).

De préférence, la force de rapprochement verticale pour rapprocher verticalement les rebords supérieur et inférieur l'un de l'autre de 10 mm, en agissant exclusivement sur les extrémités libres desdits rebords supérieur et inférieur, est inférieure à 8 N, à 6 N, à 4 N, à 3 N, à 2 N, de préférence inférieure ou égale à 1,5 N, et/ou supérieure à 0,5 N, de préférence supérieure à 1 N. Dans le mode de réalisation préféré, ce rapprochement tient donc compte de la flexibilité des arceaux, mais aussi du pivotement des rebords sur les arceaux. La force de rapprochement verticale peut être exercée sur l'extrémité libre du rebord supérieur, l'extrémité libre du rebord inférieur étant maintenue immobile et l'écarteur dentaire n'étant maintenu que par l'extrémité libre du rebord inférieur.

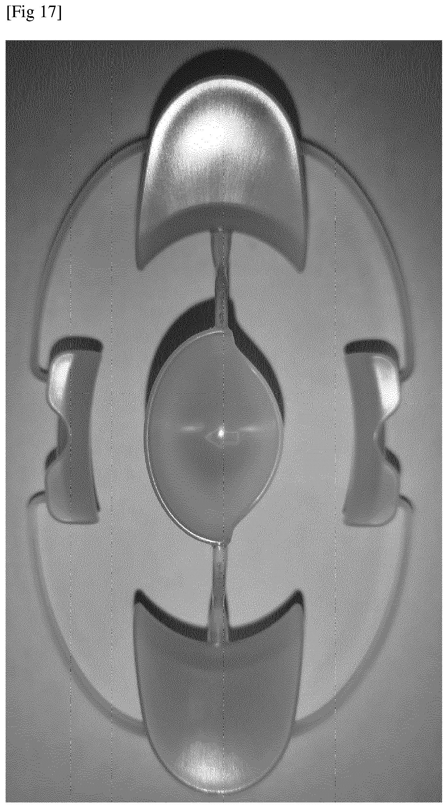

Des essais ont montré qu'avec un écarteur du type de ceux décrits dans US 6,923,761 ou WO2006014371 cités en préambule, commercialisé par la société YDM et représenté sur la figure 17, une force de rapprochement verticale de 0,6 N suffit à rapprocher de 10 mm les extrémités libres des rebords supérieur et inférieur l'une de l'autre.

Les rebords droit 14a et gauche 14b (rebords latéraux) sont fixés, de préférence rigidement, aux jonctions droite et gauche des arceaux supérieur et inférieur.

La structure 20 peut constituer un unique bloc, présentant par exemple une forme sensiblement ellipsoïdale.

La structure 20, ceinturant l'ouverture d'écarteur 50, comporte avantageusement une ouverture plus grande que l'ouverture d'écarteur 50. Ainsi, la structure 20 n'obture pas l'ouverture d'écarteur dans la position de service où la structure 20 est à l'extérieur de la bouche de l'utilisateur et fait le tour de la bouche.

De préférence et comme représenté sur l'exemple des figures 1 à 3, les rebords droit, gauche, supérieur et inférieur sont venus de matière avec la structure d'écarteur.

Chaque rebord présente de préférence la forme d'une goulotte destinée à porter une partie des lèvres de l'utilisateur, lorsque l'écarteur dentaire est en position de service.

Chaque rebord 12 présente une longueur L₁₂ ou L₁₄, mesurée en suivant le fond de la goulotte, supérieure à 1 cm, de préférence supérieure à 2 cm, de préférence supérieure à 3 cm et/ou inférieure à 15 cm, de préférence inférieure à 12 cm, de préférence inférieure à 8 cm

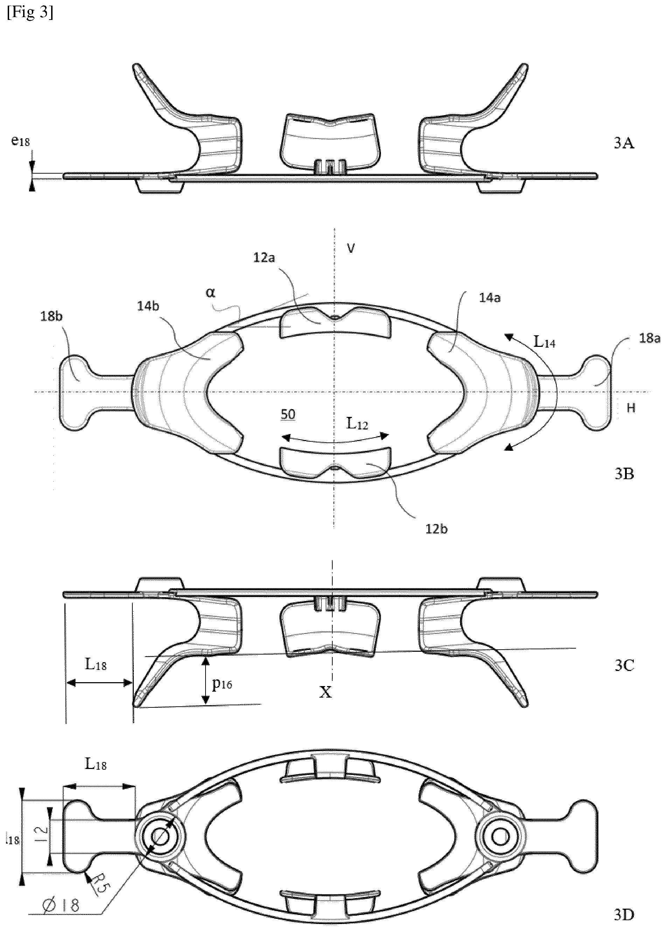

La longueur L₁₂ des rebords supérieur et inférieur est préférence la même. La longueur L₁₄ des rebords droit et gauche est préférence la même. La longueur L₁₄ des rebords latéraux est préférence supérieure à la longueur L₁₂ des rebords supérieur et inférieur (figure 3).

La longueur L₁₂ est de préférence supérieure à 1 cm, de préférence supérieure à 2 cm, de préférence supérieure à 2,5 cm, de préférence supérieure à 3 cm, et/ou inférieure à 5 cm, de préférence inférieure à 4 cm.

La longueur L₁₄ est de préférence supérieure à 3 cm, de préférence supérieure à 4 cm, de et/ou inférieure à 9 cm, de préférence inférieure à 8 cm.

Dans l'exemple de la figure 1, les rebords supérieur 12a et inférieur 12b comportent chacun, sur le bord libre de la partie du rebord destinée à être introduite dans la bouche de l'utilisateur, une encoche 42. Cette encoche présente de préférence une forme triangulaire ayant une largeur maximale L comprise entre 3 mm et 15 mm, mieux entre 5 mm et 10 mm. Alternativement, l'encoche peut présenter une forme arrondie ou rectangulaire.

L'encoche 42 est située au milieu du rebord qui la porte et permet d'améliorer le confort de l'utilisateur lorsque l'écarteur dentaire est en position de service. En effet, en position de service, l'encoche permet de laisser libre un frein labial présent au niveau des arcades supérieure et inférieure. L'amplitude de l'encoche permet en outre le déplacement de l'écarteur dentaire dans la bouche de l'utilisateur, notamment de rotations autour des arcades dentaires pour l'acquisition de photos en vues latérales.

Dans le mode de réalisation représenté, l'écarteur comporte également des oreilles droite 16a et gauche 16b agencées de manière à écarter les joues des dents. Les oreilles 16 présentent, dans l'exemple, une forme concave vers l'axe X, s'adaptant avantageusement à la forme des joues. Lesdites oreilles 16 ainsi formées permettent d'améliorer encore le confort de l'écarteur dentaire pour l'utilisateur.

Les oreilles droite 16a et gauche 16b sont de préférence de forme identique. De préférence, elles sont conformées de manière à s'étendre selon l'axe X, au-delà de la région dans laquelle s'étendent les rebords, sur une profondeur p₁₆ supérieure à 1 cm, de préférence supérieure à 1,5 cm, et/ou inférieure à 5 cm, de préférence inférieure à 4 cm, de préférence inférieure à 3 cm, de préférence inférieure à 2,5 cm (voir figure 3).

L'extrémité des oreilles est de préférence arrondie, de manière à être atraumatique.

Dans le mode de réalisation représenté, l'écarteur comporte encore des poignées droite 18a et gauche 18b, s'étendant de préférence sensiblement parallèlement au plan général de l'écarteur perpendiculaire à l'axe X d'ouverture d'écarteur. Les poignées droite 18a et gauche 18b facilitent avantageusement la manipulation de l'écarteur dentaire, en particulier lors de sa mise en position de service.

Les poignées droite 18a et gauche 18b sont de préférence de même forme. Elles présentent de préférence la forme de pattes, de préférence sensiblement plates, qui s'étendent à droite et à gauche des jonctions droite et gauche des arceaux supérieur et inférieur, respectivement.

Les poignées droite 18a et gauche 18b sont de préférence orientées vers le centre de l'ouverture d'écarteur.

De préférence, elles s'étendent sensiblement horizontalement dans la position de service.

De préférence, elles s'étendent sensiblement dans un plan parallèle au plan de l'ouverture d'écarteur, de préférence dans le plan des arceaux.

Chaque poignée présente
- une longueur L₁₈ de préférence supérieure à 1 cm, de préférence supérieure à 1,5 cm, de préférence supérieure à 2 cm, de préférence supérieure à 2,5 cm, et/ou inférieure à 10 cm, de préférence inférieure à 8 cm, de préférence inférieure à 6 cm, de préférence inférieure à 4 cm, de préférence inférieure à 3 cm, et/ou
- une largeur maximale l₁₈, de préférence à l'extrémité libre de la poignée, de préférence supérieure à 1 cm, de préférence supérieure à 1,5 cm, de préférence supérieure à 2 cm, de préférence supérieure à 2,5 cm, et/ou inférieure à 10 cm, de préférence inférieure à 8 cm, de préférence inférieure à 6 cm, de préférence inférieure à 4 cm, de préférence inférieure à 3 cm, et/ou
- une épaisseur e₁₈ de préférence supérieure à 0,1 cm, de préférence supérieure à 0,15 cm, et/ou inférieure à 0,8 cm, de préférence inférieure à 0,5 cm, de préférence inférieure à 0,4 cm, de préférence inférieure à 0,3 cm.

La longueur L₁₈ d'une poignée est mesurée à partir de la structure de l'écarteur.

Sur la figure 3, la poignée s'étend horizontalement et sa longueur est également la distance sur laquelle elle s'étend, horizontalement à partir de l'extrémité du rebord latéral le plus proche, lorsque l'écarteur est observé suivant son axe X.

Les poignées droite et gauche sont de préférence fixées, de préférence rigidement, sur les extrémités droite et gauche de la structure et/ou sur les rebords droit et gauche.

Dans un mode de réalisation préféré, les poignées droite et gauche sont venues de matière avec les rebords droit et gauche respectivement, sous la forme de pièces de jonction droite et gauche monoblocs, respectivement, sur lesquelles les extrémités droites et les extrémités gauches des arceaux, respectivement, sont fixées, de préférence rigidement (voir figure 1).

Les poignées droite et gauche sont conformées pour que l'utilisateur puisse positionner l'écarteur dentaire dans ladite position de service sans que ses doigts n'entrent en contact avec sa bouche.

Les poignées droite et gauche sont de préférence agencées de manière à s'étendre radialement à l'extérieur de l'ouverture d'écarteur, de préférence de manière à ne pas être en contact avec la peau de l'utilisateur, et en particulier avec ses joues, dans la position de service.

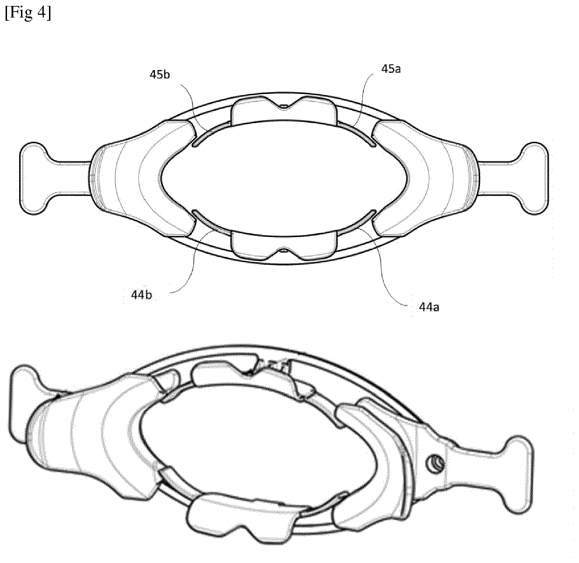

Les poignées droite et gauche permettent ainsi de préserver une bonne hygiène et sont donc particulièrement bien adaptées lorsque l'écarteur dentaire est susceptible d'être mis en position de service par l'utilisateur lui-même. Dans un mode de réalisation préféré, l'écarteur dentaire comporte également, comme représenté à la figure 4, des ailettes inférieures droite 44a et gauche 44b et supérieures droite 45a et gauche 45b.

Les ailettes inférieures 44a ; 44b et supérieures 45a ;45b sont respectivement fixées sur les rebords inférieur 12b et supérieur 12a, de préférence les ailettes sont venues de matière avec les rebords.

Sous l'effet d'une déformation de l'écarteur dentaire, notamment lors d'une torsion selon un axe horizontal ou d'une flexion autour d'un axe vertical de la structure de l'écarteur dentaire, les ailettes inférieure et supérieure droites 44a ; 45a viennent en butée contre le rebord droit 14a et les ailettes inférieure et supérieure gauches 44b ; 45b viennent en butée contre le rebord gauche 14b. Cette mise en butée limite avantageusement l'écartement des rebords supérieur et inférieur l'un de l'autre.

### Kit

Dans un mode de réalisation, l'écarteur dentaire comporte des parties d'attache d'écarteur 32 à droite et à gauche de l'ouverture d'écarteur. Les parties d'attache d'écarteur 32 sont destinées à coopérer avec des parties d'attache de support correspondantes.

Comme représenté dans la figure 1, ces parties d'attache d'écarteur peuvent être des inserts magnétiques 32a ; 32b pour la fixation du support d'écarteur sur l'écarteur dentaire. Ces inserts 32a et 32b sont respectivement portés par les rebords droit 14a et gauche 14b.

De préférence, les parties d'attache de support et les parties d'attache d'écarteur 32, et en particulier les aimant(s) et pièce(s) métallique(s), sont configurés de manière que l'écarteur dentaire ne puisse être fixé sur le support d'écarteur que dans une unique position prédéterminée.

Avantageusement, il suffit donc que l'utilisateur approche le support d'écarteur de l'écarteur dentaire pour que l'écarteur dentaire se fixe sur le support d'écarteur. L'utilisation d'une fixation magnétique permet également une fixation fiable et très précise, même lorsque l'utilisateur ne regarde pas le support d'écarteur ou l'écarteur dentaire. Ainsi, lorsque l'écarteur dentaire est en position de service, l'utilisateur peut facilement positionner le support d'écarteur sur l'écarteur dentaire, et ce de façon précise et sans aucune formation.

L'écarteur dentaire est constitué, au moins partiellement, d'élastomère.

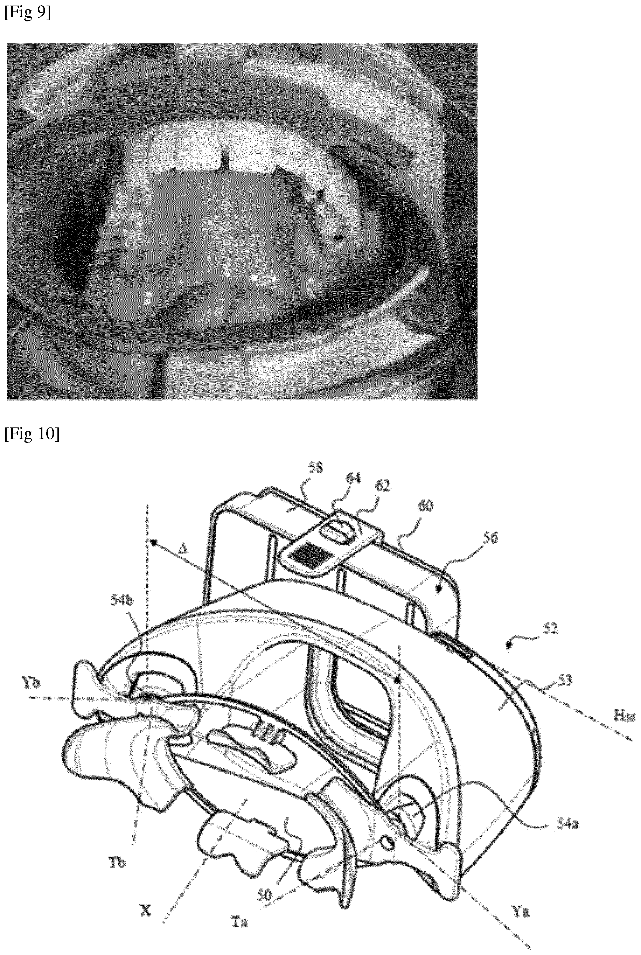

Le support d'écarteur 52, représenté sur les figures 10 et 11, présente de préférence la forme générale décrite dans la demande EP 3 391 810, de préférence la forme du support 12 décrit dans WO2020/089248. Alternativement, le support d'écarteur peut présenter la forme d'un tube ou d'un axe de liaison, par exemple un bras reliant l'appareil d'acquisition d'images à l'écarteur dentaire.

Le support d'écarteur 52 comporte de préférence un corps 53, des parties d'attache de support droite 54a et gauche 54b, fixées rigidement sur le corps 53, de préférence des aimants destinés à coopérer avec les parties d'attache droite 32a et gauche 32b de l'écarteur, respectivement, et un support pour l'appareil d'acquisition d'images, de préférence un support de téléphone mobile 56. L'écartement Δ entre les parties d'attache de support droite et gauche, réglable ou constant, de préférence constant, détermine la distance entre les rebords droit 14a et gauche 14b en position assemblée. Il détermine ainsi la rigidité de la structure de l'écarteur dans la position assemblée. De préférence, comme décrit ci-dessus, les extrémités libres des rebords supérieur et inférieur peuvent cependant se rapprocher élastiquement, par pivotement autour d'axes horizontaux respectifs.

De préférence, chaque partie d'attache de support est articulée sur le corps 53. Cette articulation permet de facilement modifier les régions de la bouche observées par l'appareil photo du téléphone mobile à travers l'ouverture d'écarteur. L'acquisition des photos par l'utilisateur en est facilitée.

Une articulation est une liaison autorisant une rotation autour d'au moins un axe de rotation.

Dans un mode de réalisation préféré, chaque partie d'attache de support est montée pivotante sur le corps 53. L'articulation est donc une liaison pivot, de préférence autour d'un axe de pivotement, de préférence horizontal dans la position de service. Elle n'autorise alors qu'une rotation autour de cet axe. De préférence, le pivotement autorise, au moins dans des positions extrêmes de pivotement basse et haute, des prises de photos du palais et de la langue, respectivement.

Les axes de pivotement des parties d'attache de support droite 54a et gauche 54b sont référencés Ya et Yb, respectivement.

L'axe de pivotement peut être fixe par rapport au support d'écarteur.

De préférence, l'axe de pivotement Ya et/ou Yb est mobile en translation par rapport au corps 53, suivant une direction différente de celle de l'axe de pivotement, de préférence suivant un axe Ta et/ou Tb horizontal, de préférence suivant un axe perpendiculaire à l'axe de pivotement. L'amplitude maximale de la translation est de préférence supérieure à 5 mm, 8 mm, 10 mm ou 12 mm et/ou inférieure à 25 mm, 20 mm, 18 mm ou 16 mm.

Lorsque l'axe de translation n'est pas perpendiculaire à l'axe de pivotement, la liaison pivot est remplacée par une liaison rotule.

Dans le mode de réalisation préféré de l'invention, dans la position montée de l'écarteur, chaque partie d'attache d'écarteur est fixée magnétiquement, de manière amovible, sur une partie d'attache de support.

Les axes Ta et Tb sont les axes selon lesquels les parties d'attache d'écarteur et de support sont attirées l'une vers l'autre, puis plaquées l'une sur l'autre. Ils sont appelés « axes d'attachement ».

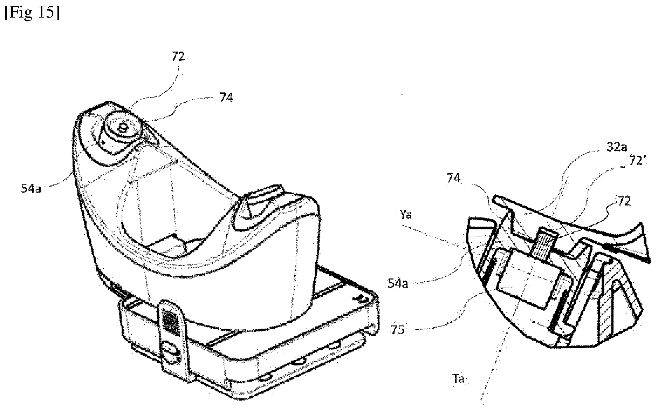

Comme illustré sur la figure 15, lesdites parties d'attache d'écarteur et de support d'une attache sont configurées pour bloquer par butée un déplacement, de préférence tout déplacement dans un plan perpendiculaire à l'axe d'attachement de cette attache.

Chaque attache comporte de préférence plusieurs butées permettant un tel blocage.

Dans le mode de réalisation de la figure 15, chaque attache comporte deux butées permettant un tel blocage, sous la forme d'un pion 72 et d'un rebord circulaire 74.

Le pion 72 représenté appartient au support, et plus précisément est rigidement fixé à un arbre 75 monté à rotation sur le support autour de l'axe de pivotement Ya de la partie d'attache de support droite 54a. Il s'étend selon l'axe d'attachement Ta et fait saillie du support de manière à pouvoir être logé, dans la position assemblée, dans un logement 72' de forme complémentaire ménagé dans la partie d'attache d'écarteur 32a. La coopération du pion et du logement permet d'assurer une butée lorsqu'une force de détachement est exercée perpendiculairement à l'axe d'attachement Ta, en particulier lorsque l'utilisateur a placé l'écarteur dans sa bouche et tire le support vers sa droite, vers sa gauche, vers le haut ou vers le bas pour l'acquisition de vues latérales ou occlusales.

Le pion 72 peut être de forme quelconque. De préférence, il est cylindrique, de préférence de section circulaire. Il est de préférence conformé pour pénétrer dans le logement sur une distance supérieure à 1 mm, de préférence supérieure à 2 mm et/ou inférieure à 15 mm, de préférence inférieure à 10 mm, de préférence inférieure à 7 mm.

Le pion peut être également tronconique afin de faciliter son insertion dans le logement.

Le rebord 74 représenté appartient au support, et plus précisément est rigidement fixé à l'arbre 75. Il est circulaire d'axe Ta et fait saillie du support de manière à définir un logement pouvant recevoir, dans la position assemblée, la partie d'attache d'écarteur 32a.

Le logement peut être également évasé, comme représenté, afin de faciliter l'insertion de la partie d'attache d'écarteur 32a.

Le rebord 74 et la partie d'attache d'écarteur 32a peuvent être complémentaires. Dans le mode de réalisation représenté, le rebord 74 définit cependant un logement légèrement plus large que la partie d'attache d'écarteur 32a qu'il reçoit. Le rebord 74 peut ainsi avantageusement guider l'utilisateur lorsqu'il assemble l'écarteur et le support, notamment pour faciliter l'introduction du pion 72 dans le logement 72'.

La hauteur du rebord, de préférence constante, est de préférence supérieure à 1 mm, de préférence supérieure à 2 mm et/ou inférieure à 15 mm, de préférence inférieure à 10 mm, de préférence inférieure à 7 mm.

La coopération du rebord 74 et de la partie d'attache d'écarteur 32a permet également d'assurer une butée lorsqu'une force de détachement est exercée perpendiculairement à l'axe d'attachement Ta, en particulier lorsque l'utilisateur a placé l'écarteur dans sa bouche et tire le support vers sa droite pour l'acquisition de photos vues de la droite.

Les axes du rebord 74 et du pion 72 sont parallèles et de préférence confondus.

Bien entendu d'autres configurations de butées sont possibles. En particulier, le pion 72 et/ou le rebord 74 pourraient appartenir à la partie d'attache d'écarteur. Les butées n'assurent pas nécessairement un blocage dans toutes les directions parallèles à un plan perpendiculaire à l'axe d'attachement. Par exemple, le rebord peut être interrompu.

Le support de téléphone mobile 56 comporte de préférence une base 58 et une porte 60, montée pivotante sur la base 58 et conformée de manière qu'un téléphone mobile, non représenté puisse être posé à plat sur la base 58, puis serré entre la base 58 et la porte 60. La porte 60 comporte une sangle de verrouillage 62, souple, qui peut être sélectivement accrochée à un pion 64 de la base 58 de manière à maintenir le téléphone mobile 66 dans cette position serrée, comme représenté sur la figure 12.

Tous les moyens connus peuvent être utilisés pour la fixation du téléphone mobile, par exemple un élastique, des bandes auto agrippantes, un clips, une vis, un volet, par exemple monté à rotation sur le support, verrouillable dans une position dans laquelle il serre l'appareil d'acquisition d'images, ou une ventouse. Cette liste d'exemples n'est pas limitative.

De préférence encore, la base est montée mobile sur le corps 53, de préférence monté mobile en translation, de préférence mobile en translation exclusivement selon une direction horizontale H₅₆, de manière à pouvoir déplacer l'axe optique du téléphone mobile par rapport à l'ouverture d'écarteur 50.

La rigidité d'ensemble de la structure de l'écarteur dentaire en position assemblée, résultant de l'écartement Δ défini entre les attaches de support, permet notamment à l'utilisateur de déplacer l'écarteur dentaire autour de l'arcade dentaire de l'utilisateur afin d'acquérir des photos pour différentes vues, en modifiant par exemple horizontalement et/ou verticalement la position de l'écarteur dentaire, de préférence en maintenant l'écarteur par les poignées 18. Ainsi, l'utilisateur peut facilement acquérir au moyen de l'appareil d'acquisition d'images, fixé sur le support d'écarteur et positionné face à l'ouverture d'écarteur, des images latérales et/ou occlusales des dents de l'utilisateur.

Dans un mode de réalisation particulier, l'appareil d'acquisition d'images est intégré dans le support d'écarteur. De préférence, l'appareil d'acquisition d'images est un téléphone mobile.

### Procédé

Avant l'étape a), dans le cas de la mise en œuvre d'un kit selon l'invention, l'appareil d'acquisition d'images, de préférence un téléphone mobile, est fixé sur le support d'écarteur, de préférence de manière amovible.

En particulier lorsque le support d'écarteur définit une chambre fermée entre le téléphone mobile et l'écarteur, le téléphone mobile est de préférence fixé sur le support d'écarteur de telle sorte que l'écran du téléphone mobile soit orienté du côté opposé à l'écarteur. Avantageusement, l'utilisateur peut ainsi, dans la position de service, visualiser cet écran, par exemple dans un miroir.

Lorsque le support d'écarteur permet à l'utilisateur de voir directement le téléphone mobile dans la position de service, le téléphone mobile est fixé de telle sorte que la caméra arrière du téléphone mobile fasse face à l'utilisateur en position de service. Avantageusement, l'utilisateur peut ainsi, dans la position de service, visualiser l'écran du téléphone mobile.

**A l'étape** a), l'utilisateur, de préférence un patient en cours de traitement orthodontique, positionne un écarteur dentaire tel que décrit précédemment, dans sa bouche.

Dans un mode de réalisation préféré, l'utilisateur rapproche les poignées droite 18a et gauche18b l'une de l'autre en tordant l'écarteur dentaire, puis introduit l'écarteur dentaire dans sa bouche en maintenant une flexion. De préférence, l'utilisateur déforme la structure faisant effectuer une flexion à la structure rigide autour d'un axe vertical passant par le centre de l'ouverture d'écarteur. Les ailettes 44a ;44b ;45a ;45b optionnelles permettent de limiter l'écartement des rebords supérieur 12a et inférieur 12b l'un de l'autre lorsque les poignées 18 sont rapprochées l'une de l'autre. L'utilisateur peut alors facilement introduire l'écarteur dentaire dans sa bouche, puis relâcher les poignées 18a et 18b pour qu'elles reviennent élastiquement dans une position proche de leur position initiale, mieux dans leur position initiale, de préférence sensiblement perpendiculaires à l'axe X, tel que représenté sur la figure 4 par exemple. L'utilisateur peut également effectuer une torsion selon un axe horizontal. L'utilisateur positionne alors d'abord un rebord latéral (rebord droit ou rebord gauche) sur la commissure correspondante, par exemple le rebord droit sur la commissure droite, puis positionne le rebord opposé sur la commissure opposée, par exemple le rebord gauche sur la commissure gauche, tout en maintenant la torsion lors du positionnement.

Alternativement, l'utilisateur introduit dans la bouche d'abord un premier des rebords droit et gauche, puis déforme l'écarteur dentaire pour y introduire le second des rebords droit et gauche.

La déformabilité d'un écarteur dentaire selon l'invention permet une insertion facilitée dans la bouche de l'utilisateur.

Dans le cas d'une mise en œuvre d'un kit selon l'invention, à **l'étape** b), l'utilisateur fixe les parties d'attache de support sur les parties d'attache d'écarteur. Selon un mode de réalisation préféré, les parties d'attache d'écarteur et de support d'écarteur forment ensemble un système aimanté, facilitant la fixation du support d'écarteur sur l'écarteur dentaire.

Alternativement ou en complément, les parties d'attache d'écarteur et de support d'écarteur comportent au moins un élastique, destiné à maintenir le support d'écarteur contre l'écarteur dentaire. Par exemple, un élastique peut être maintenu sur la structure 20 de l'écarteur dentaire, au niveau des rebords latéraux (droit et gauche), le support d'écarteur peut comporter des crochets ou une rainure destinée à recevoir l'élastique et facilitant le positionnement du support d'écarteur par rapport à l'écarteur. Alternativement et/ou additionnellement, l'utilisateur peut être guidé pour la fixation de l'écarteur dentaire au support d'écarteur. De préférence, le guidage de l'utilisateur est réalisé au moyen d'instructions visuelles et/ou sonores, par exemple émises par le téléphone mobile via une application mobile.

Les instructions sont par exemple affichées sur l'écran du téléphone mobile.

Les instructions peuvent comporter :
- des schémas illustrant différentes étapes à suivre, par exemple illustrant les différentes étapes du procédé,
- des indicateurs visuels comme des flèches explicitant les mouvements à réaliser par l'utilisateur pour acquérir des vues latérales ou occlusales par exemple,
- des points de repérage permettant de positionner l'écarteur dentaire par rapport à l'utilisateur ou l'écarteur dentaire par rapport au support d'écarteur ;
- des modèles de dent indiquant à l'utilisateur le contenu de l'image à acquérir et facilitant le positionnement du téléphone mobile par rapport à l'utilisateur, l'utilisateur pouvant par exemple superposer approximativement le modèle de dent affiché et la prévisualisation des dents de l'utilisateur apparaissant sur l'écran avant d'acquérir une image.

Alternativement, les instructions peuvent être présentées sur un support papier.

**A l'étape** c), l'utilisateur peut modifier en particulier l'angle d'acquisition par rotation du kit d'acquisition autour de la tête de l'utilisateur, tout en maintenant l'écarteur dentaire en position de service, la distance séparant l'appareil d'acquisition d'images et les dents de l'utilisateur, et/ou la position relative des mâchoires, en ouvrant ou fermant les mâchoires.

Par exemple, à partir d'une position initiale centrale, l'utilisateur fait tourner le support d'écarteur et l'écarteur dentaire autour de la tête de l'utilisateur d'un angle de plus de 15°, 20°, 30°, 45° (flèches sur la figure 12), de manière que les molaires droites fassent face à l'ouverture d'écarteur, puis de manière que les molaires gauches fassent face à l'ouverture d'écarteur. L'utilisateur peut facilement acquérir une image des molaires, grâce à la rigidité de l'écarteur dentaire en position assemblée permettant d'écarter les joues et les lèvres, sans que l'écarteur dentaire soit déformé sous l'action desdites joues et/ou desdites lèvres. De la même façon, un tel écarteur dentaire permet d'acquérir facilement des vues occlusales.

L'utilisateur peut également ouvrir ou fermer la bouche.

**A l'étape** d), l'utilisateur acquiert une ou plusieurs images, en appuyant sur le déclencheur de l'appareil d'acquisition. Alternativement, l'acquisition est effectuée automatiquement, lorsque des conditions d'acquisition sont respectées ou au bout d'une durée prédéfinie, par exemple toutes les 10 s, toutes les 5 s.

Dans un mode de réalisation préféré, l'utilisateur acquiert plusieurs images, de préférence au moins 3, de préférence au moins 5.

De préférence, les conditions d'acquisition sont modifiées entre chaque acquisition d'images. En particulier, l'utilisateur acquiert de préférence des vues mâchoires fermées, des vues mâchoires ouvertes, des vues latérales, des vues occlusales, et/ou des vues de face.

Le pion 72 et le rebord 74 assurent avantageusement une butée limitant le mouvement du support relativement à l'écarteur lorsque l'utilisateur tire le support latéralement ou verticalement pour acquérir des images de sa droite ou de sa gauche ou des images occlusales. Ils limitent ainsi le risque d'un détachement du support de l'écarteur. Lors de ces opérations.

Cependant, ils ne s'opposent pas à une traction selon les axes d'attachement Ta et Tb. L'utilisateur peut ainsi facilement désolidariser le support et l'écarteur après utilisation, en exerçant des forces tendant à les écarter suivant ces axes.

### Exemples

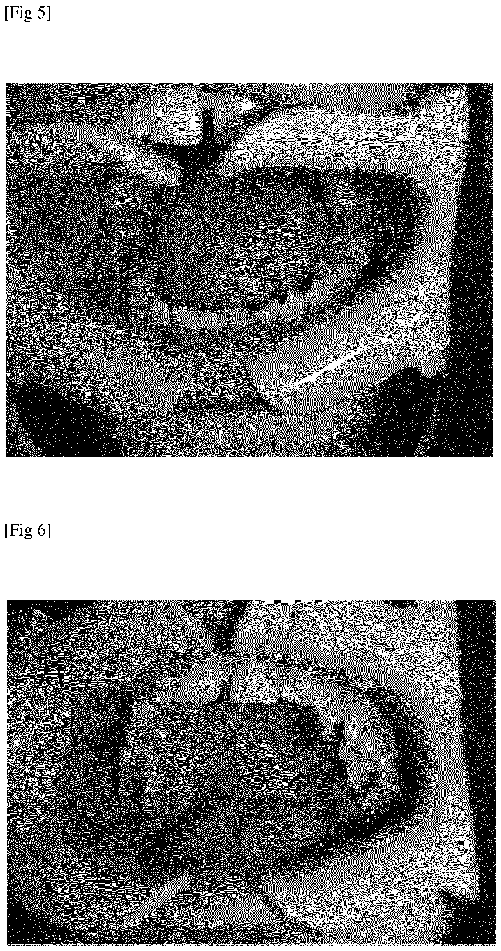

Les figures 5 et 6 sont des photos en vues occlusales résultant d'une acquisition d'images d'arcade dentaire à l'aide d'un écarteur dentaire classiquement utilisé dans l'art antérieur.

Contrairement à l'écarteur selon l'invention, l'écarteur des figures 5 et 6 n'écarte pas complétement les lèvres des dents. En particulier, sur la figure 5, on peut voir que la lèvre inférieure de l'utilisateur masque une partie des dents, notamment des incisives. Sur l'exemple de la figure 6, l'écarteur lui-même masque une partie des dents.

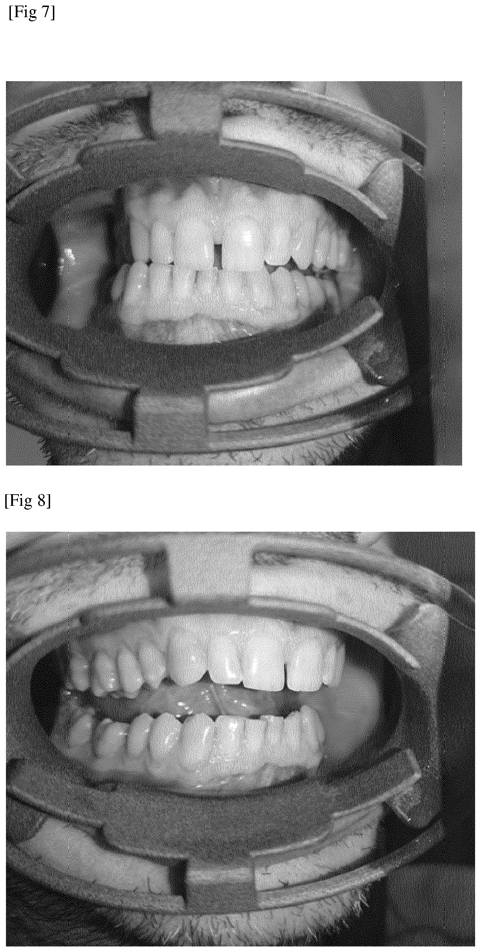

Au contraire, comme on peut le voir sur l'exemple des figures 7 à 9, les dents sont bien dégagées et visibles lors de l'acquisition d'images au moyen d'un écarteur dentaire selon l'invention. En particulier, la figure 9 illustre une vue occlusale comme la figure 6.

Comme cela apparaît clairement à présent, un écarteur dentaire selon l'invention permet de bénéficier
- d'une relative déformabilité de l'écarteur pour sa mise en position dans la bouche de l'utilisateur, puis
- d'une rigidité de la structure, éventuellement renforcée par l'attachement à un support, ce qui facilite l'observation des dents au fond de la bouche, et
- d'une déformabilité verticale, facilitant la fermeture de la bouche.

Cet écarteur est avantageusement simple, facile à utiliser et robuste, de même que le kit.

Bien entendu, l'invention n'est pas limitée aux modes de réalisation décrits ci-dessus et représentés. L'invention est définie dans les revendications annexées.

En particulier, la fixation magnétique peut être réalisée avec deux aimants ou avec un aimant et un insert métallique.

## Revendications

1. Ecarteur dentaire comportant :
- des rebords droit (14a), gauche (14b), supérieur (12a) et inférieur (12b) s'étendant autour d'une ouverture d'écarteur (50) et destinés à prendre appui, dans une position de service, sur la commissure labiale droite, la commissure labiale gauche, la partie centrale de la lèvre supérieure et la partie centrale de la lèvre inférieure d'un utilisateur, respectivement,
- une structure (20) sur laquelle sont fixés lesdits rebords, et
- des poignées droite et gauche (18a,18b) rigidement fixées à des extrémités droite et gauche de la structure, respectivement, et/ou aux rebords droit et gauche, respectivement, conformées de manière à s'étendre à l'extérieur de la bouche de l'utilisateur dans la position de service ;
**caractérisé en ce que** la structure et/ou la fixation des rebords sur la structure est/sont conformée(s) de manière que la force de rapprochement horizontale nécessaire pour rapprocher de 10 mm le rebord droit et le rebord gauche soit supérieure à 5 N,
une force de rapprochement entre deux rebords étant une force de traction exercée, parallèlement au plan de l'écarteur, sur l'extrémité libre d'un des deux rebords, pour la rapprocher élastiquement de l'extrémité libre de l'autre rebord, maintenue immobile, les directions horizontale et verticale étant définies dans la position de service dans laquelle l'écarteur est disposé sur la bouche de l'utilisateur maintenant sa tête verticalement.

2. Ecarteur dentaire selon la revendication précédente, dans lequel la force de rapprochement horizontale est supérieure ou égale à 10 N.

3. Ecarteur dentaire selon l'une quelconque des revendications précédentes, dans lequel la structure n'est comprimable horizontalement de 10 mm que sous l'action d'une force supérieure à 5 N exercée horizontalement, parallèlement au plan de l'écarteur, sur une extrémité latérale de ladite structure pour la rapprocher élastiquement de l'autre extrémité latérale de ladite structure, maintenue immobile, de préférence ladite force étant supérieure ou égale à 10 N.

4. Ecarteur dentaire selon l'une quelconque des revendications précédentes, dans lequel les poignées droite et gauche (18a,18b) sont agencées de manière à s'étendre radialement à l'extérieur de l'ouverture d'écarteur dans la position de service.

5. Ecarteur dentaire selon l'une quelconque des revendications précédentes, configuré de manière à laisser entièrement dégagée la vue à travers l'ouverture d'écarteur dans la position de service.

6. Ecarteur dentaire selon l'une quelconque des revendications précédentes, dans lequel la structure est conformée de manière à s'étendre radialement à l'extérieur de la bouche de l'utilisateur dans la position de service.

7. Ecarteur dentaire selon l'une quelconque des revendications précédentes, conformé de manière à ne s'étendre, dans la position de service, qu'à l'extérieur de la bouche de l'utilisateur et dans l'espace défini par l'extrados des dents et les lèvres de l'utilisateur.

8. Ecarteur selon l'une quelconque des revendications précédentes, les rebords supérieur (12a) et inférieur (12b) étant fixés sur la structure (20) de l'écarteur par des jonctions (24a ; 24b) autorisant un déplacement élastique vertical des extrémités libres (22a ; 22b) desdits rebords relativement à ladite structure, l'amplitude dudit déplacement étant supérieure à 10 mm, de préférence lesdites jonctions (24a ; 24b) autorisent ledit déplacement élastique sous l'effet d'une force (F_{Za}) inférieure à 10 N et supérieure à 0,5 N exercée sur l'extrémité libre supérieure (22a) du rebord supérieur (12a) ou sur l'extrémité libre inférieure (22b) du rebord inférieur (12b).

9. Ecarteur dentaire selon l'une quelconque des revendications précédentes, dans lequel la structure comporte des arceaux supérieur et inférieur se rejoignant en leurs extrémités de manière que la structure présente une forme globalement ovale, de préférence en tout point d'au moins un desdits arceaux supérieur et inférieur, la pente (α) de l'arceau est supérieure ou égale à 0° et inférieure ou égale à 60°, la pente en un point de l'arceau étant la valeur absolue du plus petit angle formé par une tangente audit arceau audit point, avec un plan horizontal dans la position de service.

10. Ecarteur dentaire selon l'une quelconque des revendications 8 à 9, dans lequel les arceaux supérieur et inférieur sont agencés de manière à s'étendre radialement à l'extérieur de l'ouverture d'écarteur dans la position de service.

11. Ecarteur dentaire selon l'une quelconque des revendications 8 à 10, les arceaux supérieur et inférieur étant configurés pour se rapprocher de plus de 10 mm sous l'action d'une force de rapprochement verticale desdits arceaux inférieure à 15 N, la force de rapprochement entre les deux arceaux étant une force de traction exercée, parallèlement au plan de l'écarteur, au milieu d'un desdits arceaux pour le rapprocher élastiquement du milieu de l'autre arceau, maintenu immobile.

12. Ecarteur dentaire selon l'une quelconque des revendications 8 à 11, les rebords supérieur et inférieur étant fixés sur des arceaux supérieur (20a) et inférieur (20b) de la structure de l'écarteur, respectivement, le rebord supérieur et/ou le rebord inférieur étant montés pivotants élastiquement sur les arceaux supérieur et inférieur, respectivement, ledit pivotement du rebord supérieur et/ou du rebord inférieur autorisant un déplacement vertical de l'extrémité libre du rebord supérieur et/ou du rebord inférieur, respectivement, d'une distance de 10 mm sous l'effet d'une force inférieure à 10 N.

13. Ecarteur dentaire selon l'une quelconque des revendications 8 à 12, dans lequel les rebords droit et gauche sont fixés rigidement à des extrémités droite et gauche, respectivement, des arceaux supérieur et inférieur.

14. Ecarteur dentaire selon l'une quelconque des revendications précédentes, le rebord supérieur et/ou le rebord inférieur comportant des ailettes droite (44a) et gauche (44b) configurées pour entrer en butée avec les rebords droit (14a) et gauche (14b), respectivement, de manière à entraver l'écartement des rebords supérieur (12a) et inférieur (12b) l'un de l'autre lors d'un rapprochement des rebords droit et gauche l'un de l'autre.

15. Ecarteur dentaire selon l'une quelconque des revendications précédentes, au moins un des rebords supérieur et inférieur comportant une encoche (42) de réception d'un frein labial.

16. Ecarteur dentaire selon l'une quelconque des revendications précédentes, la couleur d'au moins une partie de l'écarteur comportant, en code RGB, sensiblement la même valeur pour le rouge, le vert et le bleu, l'écart maximal entre les valeurs pour le rouge, le vert et le bleu étant inférieur à 10.

17. Ecarteur dentaire selon l'une quelconque des revendications précédentes, dans lequel la structure et/ou la fixation des rebords sur la structure est/sont conformée(s) de manière que la force de rapprochement « verticale » nécessaire pour rapprocher de 10 mm le rebord supérieur du rebord inférieur soit inférieure à la force de rapprochement « horizontale » nécessaire pour rapprocher de 10 mm le rebord droit et le rebord gauche, de préférence, le rapport de la force de rapprochement horizontale sur la force de rapprochement verticale étant supérieur à 5, en particulier ladite force de rapprochement horizontale étant inférieure à 20 N et ladite force de rapprochement verticale étant inférieure à 8 N.

18. Ecarteur dentaire selon l'une quelconque des revendications précédentes, dans lequel l'ouverture d'écarteur est entièrement dégagée de manière que lorsqu'on observe l'écarteur dentaire selon l'axe X de l'ouverture d'écarteur, aucune partie de l'écarteur ne masque, même partiellement, l'ouverture d'écarteur.

19. Kit comportant :
- un écarteur dentaire (10) selon l'une quelconque des revendications précédentes et comportant des parties d'attache d'écarteur (32) ; et
- un support d'écarteur (52) comportant des parties d'attache de support (54a ; 54b) disposées de manière à coopérer, de préférence magnétiquement, avec lesdites parties d'attache d'écarteur de manière à imposer, dans une position assemblée de l'écarteur sur le support d'écarteur, une distance entre les rebords droit (14a) et gauche (14b).

20. Kit selon la revendication immédiatement précédente, dans lequel dans la position assemblée, chaque partie d'attache d'écarteur est plaquée sur une partie d'attache de support correspondante suivant un axe d'attachement, lesdites parties d'attache d'écarteur et de support étant configurées pour bloquer par butée un déplacement dans un plan perpendiculaire à l'axe d'attachement.

21. Kit selon l'une quelconque des deux revendications immédiatement précédentes, comportant un téléphone mobile fixé, de manière amovible, sur le support d'écarteur dans une position permettant audit appareil d'acquisition d'images d'acquérir une image représentant l'ouverture d'écarteur (50).

22. Procédé d'acquisition d'images dentaires au moyen d'un écarteur dentaire selon l'une quelconque des revendications 1 à 18 ou d'un kit selon la revendication 19 ou 21, ledit procédé comportant les étapes suivantes :
a) mise en position de service de l'écarteur dentaire ;
b) dans le cas où le procédé est mis en œuvre au moyen d'un kit, après l'étape a), fixation du support d'écarteur à l'écarteur dentaire de façon que l'écarteur dentaire soit en position assemblée ;
c) déplacement de l'écarteur dentaire en faisant faire un mouvement de rotation à l'écarteur dentaire autour de l'utilisateur, vers la droite et/ou vers la gauche de l'utilisateur, de façon à rendre au moins une molaire visible à travers l'ouverture d'écarteur, et/ou
fermeture de la bouche de l'utilisateur de façon à rendre visibles, à travers l'ouverture d'écarteur, des dents en position bouche fermée ;
d) acquisition, au moyen d'un appareil d'acquisition d'images, d'au moins une image représentant ladite molaire et/ou représentant des dents en position bouche fermée.

23. Procédé selon la revendication précédente, dans lequel, avant l'étape d), l'appareil d'acquisition d'images est fixé sur le support d'écarteur dans une position permettant audit appareil d'acquisition d'images d'acquérir une image représentant l'ouverture d'écarteur (50).

## Patentansprüche

1. Dentaler Abstandshalter, umfassend:
- einen rechten (14a), einen linken (14b), einen oberen (12a) und einen unteren (12b) Rand, die sich um eine Abstandshalteröffnung (50) herum erstrecken und dazu bestimmt sind, in einer Betriebsposition an dem rechten Mundwinkel, dem linken Mundwinkel, dem mittleren Teil der Oberlippe beziehungsweise dem mittleren Teil der Unterlippe eines Benutzers anzuliegen,
- eine Struktur (20), an der die Ränder befestigt sind, und
- einen rechten und einen linken Griff (18a, 18b), die an dem rechten beziehungsweise dem linken Ende der Struktur und/oder an dem rechten beziehungsweise dem linken Rand starr befestigt sind, die so geformt sind, dass sie sich in der Betriebsposition aus dem Mund des Benutzers heraus erstrecken;
**dadurch gekennzeichnet, dass**
die Struktur und/oder die Befestigung der Ränder an der Struktur so geformt ist/sind, dass die horizontale Annäherungskraft, die zum Annähern des rechten Rands und des linken Rands um 10 mm erforderlich ist, größer als 5 N ist, eine Annäherungskraft zwischen zwei Rändern eine Zugkraft ist, die parallel zu der Ebene des Abstandshalters auf das freie Ende eines der zwei Ränder ausgeübt wird, zum elastischen Annähern davon an das freie Ende des anderen Rands, das ruhig gehalten wird, wobei die horizontale und die vertikale Richtung in der Betriebsposition definiert sind, in der der Abstandshalter auf dem Mund des Benutzers platziert ist, der seinen Kopf vertikal hält.

2. Dentaler Abstandshalter nach dem vorstehenden Anspruch, wobei die horizontale Annäherungskraft größer als oder gleich 10 N ist.

3. Dentaler Abstandshalter nach einem der vorstehenden Ansprüche, wobei die Struktur nur unter Einwirkung einer Kraft von mehr als 5 N, die horizontal, parallel zu der Ebene des Abstandshalters, auf ein seitliches Ende der Struktur zum elastischen Annähern davon an das andere seitliche Ende der Struktur ausgeübt wird, das ruhig gehalten wird, um 10 mm horizontal zusammendrückbar ist, wobei vorzugsweise die Kraft größer als oder gleich 10 N ist.

4. Dentaler Abstandshalter nach einem der vorstehenden Ansprüche, wobei der rechte und der linke Griff (18a, 18b) so angeordnet sind, dass sie sich in der Betriebsposition radial aus der Abstandshalteröffnung heraus erstrecken.

5. Dentaler Abstandshalter nach einem der vorstehenden Ansprüche, der so konfiguriert ist, dass er in der Betriebsposition die Sicht durch die Abstandshalteröffnung völlig frei lässt.

6. Dentaler Abstandshalter nach einem der vorstehenden Ansprüche, wobei die Struktur so geformt ist, dass sie sich in der Betriebsposition radial aus dem Mund des Benutzers heraus erstreckt.

7. Dentaler Abstandshalter nach einem der vorstehenden Ansprüche, der so geformt ist, dass er sich in der Betriebsposition nur aus dem Mund des Benutzers heraus und in dem Raum erstreckt, der durch die Oberseite der Zähne und der Lippen des Benutzers definiert wird.

8. Abstandshalter nach einem der vorstehenden Ansprüche, wobei der obere (12a) und der untere (12b) Rand durch Verbindungsstellen (24a; 24b) an der Struktur (20) des Abstandshalters befestigt sind, die eine vertikale elastische Verschiebung der freien Enden (22a; 22b) der Ränder relativ zu der Struktur ermöglichen, wobei die Amplitude der Verschiebung größer als 10 mm ist, vorzugsweise die Verbindungsstellen (24a; 24b) die elastische Verschiebung unter Einwirkung einer Kraft (F_{Za}) von weniger als 10 N und mehr als 0,5 N ermöglichen, die auf das obere freie Ende (22a) des oberen Rands (12a) oder auf das untere freie Ende (22b) des unteren Rands (12b) ausgeübt wird.

9. Dentaler Abstandshalter nach einem der vorstehenden Ansprüche, wobei die Struktur einen oberen und einen unteren Bogen umfasst, die an ihren Enden so zusammentreffen, dass die Struktur eine im Allgemeinen ovale Form aufweist, vorzugsweise an jedem Punkt von mindestens einem des oberen und des unteren Bogens die Steigung (α) des Bogens größer als oder gleich 0° und kleiner als oder gleich 60° ist, wobei die Steigung an einem Punkt des Bogens der Absolutwert des kleinsten Winkels ist, der durch eine Tangente an dem Bogen an dem Punkt ausgebildet wird, wobei eine horizontale Ebene in der Betriebsposition ist.

10. Dentaler Abstandshalter nach einem der Ansprüche 8 bis 9, wobei der obere und der untere Bogen so angeordnet sind, dass sie sich in der Betriebsposition radial aus der Abstandshalteröffnung heraus erstrecken.

11. Dentaler Abstandshalter nach einem der Ansprüche 8 bis 10, wobei der obere und der untere Bogen zum sich Annähern um mehr als 10 mm unter der Einwirkung einer vertikalen Annäherungskraft der Bögen konfiguriert sind, die weniger als 15 N beträgt, wobei die Annäherungskraft zwischen den zwei Bögen eine Zugkraft ist, die parallel zu der Ebene des Abstandshalters auf die Mitte eines der Bögen zum elastischen Annähern davon an die Mitte des anderen Bogens ausgeübt wird, der ruhig gehalten wird.

12. Dentaler Abstandshalter nach einem der Ansprüche 8 bis 11, wobei der obere und der untere Rand jeweils an dem oberen (20a) und dem unteren (20b) Bogen der Struktur des Abstandshalters befestigt sind, wobei der obere Rand und/oder der untere Rand schwenkbar elastisch an dem oberen beziehungsweise dem unteren Bogen montiert sind, wobei das Schwenken des oberen Rands und/oder des unteren Rands eine vertikale Verschiebung des freien Endes des oberen Rands und/oder des unteren Rands jeweils um einen Abstand von 10 mm unter Einwirkung einer Kraft von weniger als 10 N ermöglicht.

13. Dentaler Abstandshalter nach einem der Ansprüche 8 bis 12, wobei der rechte und der linke Rand an dem rechten beziehungsweise dem linken Ende des oberen und des unteren Bogens starr befestigt sind.

14. Dentaler Abstandshalter nach einem der vorstehenden Ansprüche, wobei der obere Rand und/oder der untere Rand eine rechte (44a) und eine linke (44b) Rippe umfasst, die zum Inanschlagkommen jeweils mit dem rechten (14a) und dem linken (14b) Rand konfiguriert sind, um den Abstand des oberen (12a) und des unteren (12b) Rands zueinander bei einer Annäherung des rechten und des linken Rands zueinander zu erschweren.

15. Dentaler Abstandshalter nach einem der vorstehenden Ansprüche, wobei mindestens einer des oberen und des unteren Rands eine Kerbe (42) zum Aufnehmen eines Lippenbändchens aufweist.

16. Dentaler Abstandshalter nach einem der vorstehenden Ansprüche, wobei die Farbe mindestens eines Teils des Abstandshalters in dem RGB-Code im Wesentlichen den gleichen Wert für rot, grün und blau aufweist, wobei der maximale Unterschied zwischen den Werten für rot, grün und blau weniger als 10 beträgt.

17. Dentaler Abstandshalter nach einem der vorstehenden Ansprüche, wobei die Struktur und/oder die Befestigung der Ränder an der Struktur so geformt ist/sind, dass die "vertikale" Annäherungskraft, die zum Annähern des oberen Rands um 10 mm an den unteren Rand erforderlich ist, kleiner als die "horizontale" Annäherungskraft ist, die zum Annähern des rechten Rands und des linken Rands um 10 mm erforderlich ist, wobei vorzugsweise das Verhältnis der horizontalen Annäherungskraft zu der vertikalen Annäherungskraft größer als 5 ist, wobei insbesondere die horizontale Annäherungskraft weniger als 20 N und die vertikale Annäherungskraft weniger als 8 N beträgt.

18. Dentaler Abstandshalter nach einem der vorstehenden Ansprüche, wobei die Öffnung des Abstandshalters völlig durchscheinend ist, sodass, wenn der dentale Abstandshalter entlang der Achse X der Abstandshalteröffnung betrachtet wird, kein Teil des Abstandshalters die Abstandshalteröffnung verdeckt, auch nicht teilweise.

19. Kit, umfassend:
- einen dentalen Abstandshalter (10) nach einem der vorstehenden Ansprüche und der Abstandshalteranbringungsteile (32) umfasst; und
- einen Abstandhalterträger (52), der Trägeranbringungsteile (54a; 54b) umfasst, die so angeordnet sind, dass sie, vorzugsweise magnetisch, mit den Abstandshalteranbringungsteilen zusammenwirken, um in einer zusammengesetzten Position des Abstandhalters auf dem Abstandhalterträger einen Abstand zwischen dem rechten (14a) und dem linken (14b) Rand aufzuerlegen.

20. Kit nach dem unmittelbar vorstehenden Anspruch, wobei in der zusammengesetzten Position jedes Abstandshalteranbringungsteil entlang einer Anbringungsachse auf ein entsprechendes Trägeranbringungsteil gedrückt wird, wobei die Abstands- und Trägeranbringungsteile zum Blockieren einer Verschiebung in einer Ebene senkrecht zu der Anbringungsachse durch einen Anschlag konfiguriert sind.

21. Kit nach einem der zwei unmittelbar vorstehenden Ansprüche, umfassend ein Mobiltelefon, das abnehmbar an dem Abstandshalterträger in einer Position befestigt ist, die es zulässt, dass die Bilderfassungseinrichtung ein Bild erfasst, das die Abstandshalteröffnung (50) darstellt.

22. Verfahren zum Erfassen von dentalen Bildern mittels eines dentalen Abstandshalters nach einem der Ansprüche 1 bis 18 oder eines Kits nach Anspruch 19 oder 21, wobei das Verfahren die folgenden Schritte umfasst:
a) Positionieren des dentalen Abstandshalters in der Betriebsposition;
b) für den Fall, dass das Verfahren mittels eines Kits durchgeführt wird, nach Schritt a) Befestigen des Abstandshalterträgers an dem dentalen Abstandshalter, so dass der dentale Abstandshalter in der zusammengesetzten Position ist;
c) Verschieben des Zahnabstandshalters durch Bewirken einer Drehbewegung des Zahnabstandshalters um den Benutzer herum nach rechts und/oder nach links von dem Benutzer, um mindestens einen Backenzahn durch die Abstandshalteröffnung sichtbar zu machen, und/oder
Schließen des Mundes des Benutzers, um die Zähne in der Position mit geschlossenem Mund durch die Abstandshalteröffnung sichtbar zu machen;
d) Erfassen mittels einer Bilderfassungseinrichtung von mindestens einem Bild, das den Backenzahn und/oder die Zähne in der Position mit geschlossenem Mund darstellt.

23. Verfahren nach dem vorstehenden Anspruch, wobei vor Schritt d) die Bilderfassungseinrichtung auf dem Abstandshalterträger in einer Position befestigt wird, die es zulässt, dass die Bilderfassungseinrichtung ein Bild erfasst, das die Abstandshalteröffnung (50) darstellt.

## Claims

1. Dental retractor comprising:
- a right flange (14a), left flange (14b), upper flange (12a) and lower flange (12b) extending around a retractor opening (50) and intended to rest, in a service position, on the right labial commissure, the left labial commissure, the central part of the upper lip and the central part of the lower lip of a user, respectively,
- a structure (20) on which said flanges are attached, and
- right and left handles (18a, 18b) which are rigidly attached to right and left ends of the structure, respectively, and/or to the right and left flanges, respectively, which handles are shaped so as to extend outside the user's mouth in the service position;
**characterized in that**
the structure and/or the attachment of the flanges to the structure is/are shaped such that the horizontal closing force required to bring the right flange and left flange 10 mm closer together is greater than 5 N, a closing force between two flanges being a tensile force which is exerted, parallel to the plane of the retractor, on the free end of one of the two flanges, to bring it elastically closer to the free end of the other flange, held stationary, the horizontal and vertical directions being defined in the service position in which the retractor is placed over the user's mouth, holding their head vertically.

2. Dental retractor according to the preceding claim, wherein the horizontal closing force is greater than or equal to 10 N.

3. Dental retractor according to any one of the preceding claims, wherein the structure is horizontally compressible by 10 mm only under the action of a force greater than 5 N exerted horizontally, parallel to the plane of the retractor, on one lateral end of said structure to bring it elastically closer to the other lateral end of said structure, which is held stationary, preferably said force being greater than or equal to 10 N.

4. Dental retractor according to any one of the preceding claims, wherein the right and left handles (18a, 18b) are arranged so as to extend radially outside the retractor opening in the service position.

5. Dental retractor according to any one of the preceding claims, configured so as to leave the view through the retractor opening completely unobstructed in the service position.

6. Dental retractor according to any one of the preceding claims, wherein the structure is shaped so as to extend radially outside the user's mouth in the service position.

7. Dental retractor according to any one of the preceding claims, shaped so as to extend, in the service position, only outside the user's mouth and into the space defined by the extrados of the user's teeth and lips.

8. Retractor according to any one of the preceding claims, the upper flange (12a) and lower flange (12b) being attached to the structure (20) of the retractor by joints (24a; 24b) allowing vertical elastic movement of the free ends (22a; 22b) of said flanges relative to said structure, the amplitude of said movement being greater than 10 mm, preferably said joints (24a; 24b) allow said elastic movement under the effect of a force (F_{Za}) of less than 10 N and greater than 0.5 N exerted on the upper free end (22a) of the upper flange (12a) or on the lower free end (22b) of the lower flange (12b).

9. Dental retractor according to any one of the preceding claims, wherein the structure comprises an upper arch and lower arch meeting at the ends thereof so that the structure has a generally oval shape, preferably at any point of at least one of said upper arch and lower arch the slope (α) of the arch is greater than or equal to 0° and less than or equal to 60°, the slope at a point of the arch being the absolute value of the smallest angle formed by a tangent to said arch at said point, with a horizontal plane in the service position.

10. Dental retractor according to any one of claims 8 to 9, wherein the upper arch and lower arch are arranged so as to extend radially outside the retractor opening in the service position.

11. Dental retractor according to any one of claims 8 to 10, the upper arch and lower arch being configured to move closer together by more than 10 mm under the action of a vertical closing force of said arches of less than 15 N, the closing force between the two arches being a tensile force which is exerted, parallel to the plane of the retractor, at the middle of one of said arches to move it elastically closer to the middle of the other arch, which is held stationary.

12. Dental retractor according to any one of claims 8 to 11, the upper flange and lower flange being attached to the upper arch (20a) and lower arch (20b) of the retractor structure, respectively, the upper flange and/or the lower flange being elastically pivotably mounted on the upper arch and lower arch, respectively, said pivoting of the upper flange and/or the lower flange allowing vertical movement of the free end of the upper flange and/or the lower flange, respectively, by a distance of 10 mm under the effect of a force of less than 10 N.

13. Dental retractor according to any one of claims 8 to 12, wherein the right flange and left flange are rigidly attached to right and left ends, respectively, of the upper arch and lower arch.

14. Dental retractor according to any one of the preceding claims, the upper flange and/or the lower flange comprising a right fin (44a) and left fin (44b) configured to abut the right flange (14a) and left flange (14b), respectively, so as to impede the upper flange (12a) and lower flange (12b) from moving away from one another when the right flange and left flange are moved towards one another.

15. Dental retractor according to any one of the preceding claims, at least one of the upper flange and lower flange comprising a notch (42) for receiving a labial frenulum.

16. Dental retractor according to any one of the preceding claims, the color of at least one part of the retractor comprising, in RGB code, substantially the same value for red, green and blue, the maximum deviation between the values for red, green and blue being less than 10.

17. Dental retractor according to any one of the preceding claims, wherein the structure and/or the attachment of the flanges to the structure is/are shaped such that the "vertical" closing force required to bring the upper flange 10 mm closer to the lower flange is less than the "horizontal" closing force required to bring the right flange 10 mm closer to the left flange, preferably, the ratio of the horizontal closing force to the vertical closing force being greater than 5, in particular said horizontal closing force being less than 20 N and said vertical closing force being less than 8 N.

18. Dental retractor according to any one of the preceding claims, wherein the retractor opening is completely unobstructed such that when the dental retractor is viewed along the X axis of the retractor opening, no part of the retractor even partially obscures the retractor opening.

19. Kit comprising:
- a dental retractor (10) according to any one of the preceding claims and comprising retractor attachment parts (32); and
- a retractor support (52) comprising support attachment parts (54a; 54b) arranged so as to engage, preferably magnetically, with said retractor attachment parts so as to impose, in an assembled position of the retractor on the retractor support, a distance between the right flange (14a) and left flange (14b).

20. Kit according to the immediately preceding claim, wherein in the assembled position, each retractor attachment part is pressed onto a corresponding support attachment part along an attachment axis, said retractor and support attachment parts being configured to block, by means of abutment, movement in a plane perpendicular to the attachment axis.

21. Kit according to any one of the two immediately preceding claims, comprising a cell phone removably attached to the retractor support in a position allowing said image acquisition device to acquire an image representing the retractor opening (50).

22. Method of acquiring dental images by means of a dental retractor according to any one of claims 1 to 18 or a kit according to claim 19 or 21, said method comprising the following steps:
a) positioning the dental retractor in the service position;
b) if the method is implemented using a kit, after step a), attaching the retractor support to the dental retractor so that the dental retractor is in the assembled position;
c) moving the dental retractor by rotating the dental retractor around the user, to the right and/or left of the user, so as to make at least one molar visible through the retractor opening, and/or
closing the user's mouth so that teeth in the closed-mouth position are visible through the retractor opening;
d) acquiring, by means of an image acquisition device, at least one image representing said molar and/or representing teeth in a closed-mouth position.

23. Method according to the preceding claim, wherein, prior to step d), the image acquisition device is attached to the retractor support in a position allowing said image acquisition device to acquire an image representing the retractor opening (50).
